# EUROPEAN PATENT APPLICATION

(11) **EP 4 342 979 A1**
(43) Date of publication of application: **27.03.2024**
(21) Application number: 22196546.0
(22) Date of filing: 20.09.2022
(51) Int. Cl.: C12N 5/071, A61K 35/12, C12N 15/62, C12N 15/90

(54) **NOVEL METHOD**

(71) Applicant: Bit Bio Discovery GmbH, 1110 Wien (AT)
(72) Inventor: The designation of the inventor has not yet been filed
(74) Representative: Sagittarius IP

(57) **Abstract**

The invention relates to methods of forward programming an induced pluripotent stem cell (iPSC) into a somatic cell, said methods comprising at least dual targeting of safe harbour sites in the genome of an iPSC. The invention also includes cells obtained by such methods.

## Description

### FIELD OF THE INVENTION

The present invention relates to methods of forward programming an induced pluripotent stem cell (iPSC) into a somatic cell, said methods comprising at least dual targeting of safe harbour sites in the genome of an iPSC (genomic safe harbour sites; GSHs), with the system for induced transcription split over two or more GSH sites. In the methods of the invention, one GSH site is modified to contain a transcriptional regulator that is required to induce transcription of the genetic sequence contained within the inducible cassette inserted into a different GSH site elsewhere in the genome. In certain embodiments, the forward programming utilises CRISPR activation (CRISPRa) of endogenous lineage-specific factor genes. Also provided is a method for forward programming an iPSC into a neuron, as well as cells (e.g., forward programmed somatic cells) and neurons obtained by the methods, and their use in therapy and the treatment of diseases/disorders.

### BACKGROUND

The isolation or *in vitro* differentiation of many human cell types remains a challenging and inefficient procedure. Conventional differentiation protocols require developmental intermediate states and different culture conditions, generating mixed subtypes of cells with immature phenotypes and with varying degrees of efficiency. In contrast, the direct conversion of pluripotent stem cells to other cell types by forced overexpression of transcription factors holds great promise to overcome these lengthy and challenging procedures.

CRISPR activation (CRISPRa) uses a catalytically inactive Cas protein (*e.g*., Cas9, wherein the catalytically inactive version is referred to as "dCas9") fused to one or more transcriptional activator proteins with the ability to activate the expression of endogenous target genes (*e.g*., "VPR", a fusion of the VP64, p65, and Rta transcriptional activators). Like for CRISPR/Cas9, the CRISPR effector is guided to the target site by a complementary guide RNA (gRNA). After binding, the transcriptional activator domain(s) recruit important co-factors as well as RNA polymerases for transcription of the targeted gene.

In recent years, CRISPRa-based cell programming has been demonstrated successfully by a number of academic groups. By targeting the genes of known developmental transcription factors like NEUROG2 or NEUROD1 using a catalytically inactive programmable nuclease fused to transcription activator proteins (*e.g*., dCas9-VPR) with a lentiviral pool of gRNAs, hiPSCs were rapidly differentiated into cells with neuronal morphology (Chavez et al. (2015) Nat. Methods. 12(4):326-328, doi: 10.1038/ nmeth.3312).

However, the lentiviral delivery of gRNAs or transgenes required for CRISPRa bears several shortcomings. Most importantly, its random integration pattern can lead to varying expression levels of the delivered gRNA, depending on where the lentivirus integrates. In addition, lentiviruses are recognised as foreign elements and are subject to silencing, which can occur by various mechanisms including activation-induced cytidine deamination and other epigenetic regulatory mechanisms.

There is therefore a need to develop methods and tools which overcome these constraints by providing predictable intracellular delivery of gRNAs and/or transgenes (*e.g*., expression cassettes) and efficient expression levels.

### SUMMARY OF THE INVENTION

According to a first aspect of the invention, there is provided a method of forward programming an induced pluripotent stem cell (iPSC) into a somatic cell, said method comprising:
(i) targeted insertion of a gene encoding a transcriptional regulator protein into a first genomic safe harbour (GSH) site;
(ii) targeted insertion of an inducible cassette into a second GSH site, wherein said inducible cassette comprises a genetic sequence encoding a catalytically inactive programable nuclease protein and one or more transcription activator proteins operably linked to an inducible promoter, and wherein said inducible promoter is regulated by the transcriptional regulator protein;
(iii) targeted insertion of one or more guide RNA (gRNA) sequences into a third GSH site, wherein said one or more gRNA sequences are complementary to one or more transcription start sites (TSSs) of an endogenous lineage-specific factor gene and are operably linked to a constitutive promoter,
wherein the first, second and third GSH sites are different.

In another aspect, there is provided a method of forward programming an iPSC into a neuron, said method comprising:
(i) targeted insertion of a gene encoding a transcriptional regulator protein into a first genomic safe harbour (GSH) site, wherein the first GSH site is the ROSA26 locus;
(ii) targeted insertion of an inducible cassette into a second GSH site, wherein said inducible cassette comprises a genetic sequence encoding a catalytically inactive programmable nuclease protein and one or more transcription activator proteins operably linked to an inducible promoter, wherein said inducible promoter is regulated by the transcriptional regulator protein, and wherein the second GSH site is the AAVS1 locus; and
(iii) targeted insertion of one or more guide RNA (gRNA) sequences into a third GSH site, wherein said one or more gRNA sequences are complementary to one or more transcription start sites (TSSs) of the endogenous *NEUROG2* gene and/or the TSS of the endogenous *NEUROD1* gene and is operably linked to a constitutive promoter, and wherein the third GSH site is the CLYBL gene.

According to a further aspect of the invention, there is provided a cell obtained by the methods described herein.

In a yet further aspect, there is provided a cell with a modified genome that comprises:
(i) an inserted genetic sequence encoding a transcriptional regulator protein at a first genomic safe harbour (GSH) site;
(ii) an inserted inducible cassette comprising a genetic sequence encoding a catalytically inactive programmable nuclease protein and one or more transcription activator proteins operably linked to an inducible promoter at a second GSH site, wherein said inducible promoter is regulated by the transcriptional regulator protein; and
(iii) an inserted genetic sequence encoding one or more gRNA sequences operably linked to a constitutive promoter at a third GSH site, wherein said one or more gRNA sequences are complementary to one or more transcription start sites (TSSs) of an endogenous lineage-specific factor gene,
wherein the first, second and third GSH sites are different.

In another aspect, there is provided a neuron forward programmed from an iPSC with a modified genome that comprises:
(i) an inserted genetic sequence encoding a transcriptional regulator protein at a first genomic safe harbour (GSH) site, wherein the first GSH site is the ROSA26 locus;
(ii) an inserted inducible cassette comprising a genetic sequence encoding a catalytically inactive programmable nuclease protein and one or more transcription activator proteins operably linked to an inducible promoter at a second GSH site, wherein said inducible promoter is regulated by the transcriptional regulator protein, and wherein the second GSH site is the AAVS1 locus; and
(iii) an inserted genetic sequence encoding one or more guide RNA (gRNA) sequences operably linked to a constitutive promoter at a third GSH site, wherein said one or more gRNA sequences are complementary to one or more transcription start sites (TSSs) of the endogenous *NEUROG2* gene and/or the TSS of the endogenous *NEUROD1* gene, and wherein the third GSH site is the CLYBL gene.

According to a yet further aspect of the invention, there is provided the cell as described herein for use in therapy.

In a still further aspect, there is provided the cell as described herein for use in the treatment of cancer, a neurological disorder, an inflammatory disease, an autoimmune disease and/or a chronic infectious disease.

### BRIEF DESCRIPTION OF THE FIGURES

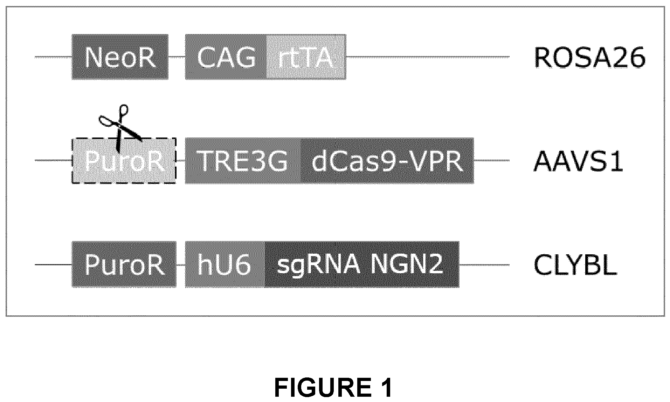

**Figure 1****:** Cell line engineering strategy for single gRNA insertion (Example 1). Knock-in of CAG driven rtTA into the ROSA26 locus, doxycycline-inducible dCas9-VPR into the AAVS1 locus and hU6-gRNA NGN2 cassette into the CLYBL locus.

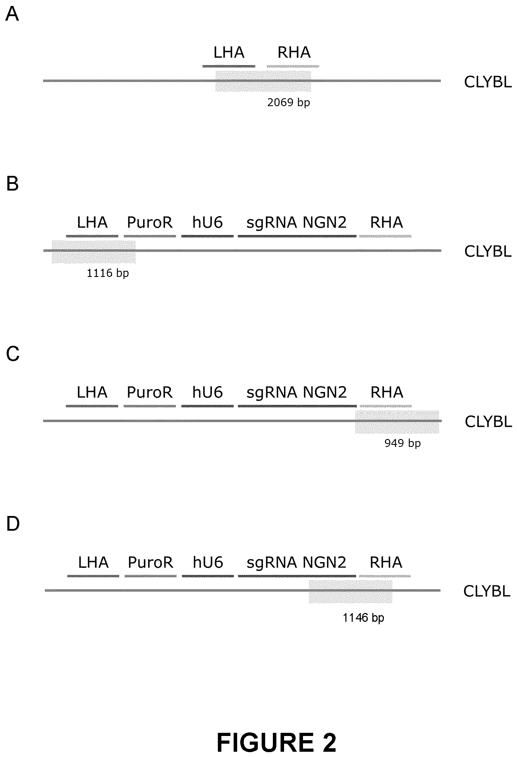

**Figure 2****:** Schematic representations of the hU6-sgRNA cassette regions spanned by genotyping primers and the expected PCR products for single gRNA insertion embodiments of the invention (Example 1). Expected products of: PR2284 and PR2264 primers when no integration has occurred (2069 bp; **A**), PR2284 and PR2285 primers from the 5' end of the hU6-sgRNA cassette when integration as occurred (1116 bp; **B**); PR2282 and PR2149 primers from the 3' end of the hU6-sgRNA cassette when integration as occurred (949 bp; **C**); PR2079 and PR2232 primers from on-target integration (1146 bp; **D**).
**Figure 3****:** CRISPRa-based cell programming of iPSCs into neuronal cells using single gRNA insertion (Example 1) with a gRNA expression cassette integrated into the CLYBL safe harbour locus.
**Figure 4****:** Immunofluorescence staining of neurons at day 7 of forward programming using single gRNA insertion (Example 1). Cells were stained for MAP2.
**Figure 5****:** Schematic representation of the hU6 promoter driven Csy4-cleavable guide array with two gRNAs for multiple gRNA insertion embodiments of the invention (Example 2).
**Figure 6****:** Cell line engineering strategy for multiple gRNA insertion embodiments of the invention (Example 2). Knock-in of CAG driven rtTA into the ROSA26 locus and doxycycline-inducible Csy4-dCas9-VPR into the AAVS1 locus.
**Figure 7****:** Schematic representations of the Csy4-cleavable guide arrays for multiple gRNA insertion embodiments of the invention (Example 2). Array 1 contains only one gRNA targeting upstream of the transcription start site (TSS) of *NEUROG2;* Array 2 encodes two gRNAs of which one is a non-targeting guide and the second guide targets upstream of the TSS of *NEUROG2* (same as array 1); Array 3 consists of two guides, both targeting different positions upstream of the TSS of *NEUROG2.*
**Figure 8****:** Brightfield images of day 7 glutamatergic neurons forward programmed using a multiple gRNA array (Example 2).
**Figure 9****:** Immunofluorescence staining of neurons at day 7 of forward programming using a multiple gRNA array (Example 2). Cells were stained for MAP2 and DAPI. GFP positive cells are transduced with lentivirus and harbour the indicated Csy4-guide array.

### DETAILED DESCRIPTION OF THE INVENTION

According to a first aspect of the invention, there is provided a method of forward programming an induced pluripotent stem cell (iPSC) into a somatic cell, said method comprising:
(i) targeted insertion of a gene encoding a transcriptional regulator protein into a first genomic safe harbour (GSH) site;
(ii) targeted insertion of an inducible cassette into a second GSH site, wherein said inducible cassette comprises a genetic sequence encoding a catalytically inactive programable nuclease protein and one or more transcription activator proteins operably linked to an inducible promoter, and wherein said inducible promoter is regulated by the transcriptional regulator protein;
(iii) targeted insertion of one or more guide RNA (gRNA) sequences into a third GSH site, wherein said one or more gRNA sequences are complementary to one or more transcription start sites (TSSs) of an endogenous lineage-specific factor gene and are operably linked to a constitutive promoter,
wherein the first, second and third GSH sites are different.

Thus, described herein is a method of activating an endogenous gene in a cell, such as a lineage-specific factor gene in an iPSC, by inserting the machinery required for said activation into genomic safe harbour (GSH) sites. Such activation may utilise CRISPR activation (CRISPRa), with insertion of an inducible cassette comprising a genetic sequence encoding a catalytically inactive programmable Cas nuclease (e.g., dCas9 or dCas12a) and one or more transcription activator proteins into a GSH site (e.g., a second GSH site), and insertion of one or more guide RNA (gRNA) sequences complementary to one or more transcription start sites (TSSs) of an endogenous lineage-specific factor gene into a further GSH site (e.g., a third GSH site), wherein the GSHs are different. The method is therefore particularly applicable to the forward programming of an iPSC into a somatic cell without the need for exogenous lineage-specific factors or sequences encoding them.

### Genomic Safe Harbour (GSH) Sites & Insertions Therein

The methods described herein are based upon the at least dual targeting of safe harbour sites in the genome of an iPSC, with the system for induced transcription split over two or more GSH sites. However, this method is not limited to stem cells, and can be used to modify the genome of any cell type, for example in research or in gene therapy or in the production of biologics such as antibodies or cytokines. In the methods of the invention one GSH site is modified to contain a transcriptional regulator that is required to induce transcription of the genetic sequence contained within the inducible cassette inserted into a different GSH site elsewhere in the genome. The transcriptional regulator is preferably constitutively expressed. It is preferred that an exogenous substance/agent has to be supplied in order to control the activity of the transcriptional regulator protein and thus control expression of the inducible cassette. Since at least two separate GSH sites are used in the method of the invention, there are a total of four possible insertion loci, since each GSH site exists on both chromosomes of a diploid organism. This increases the amount of transcription possible from the cell if all four loci are modified using the method of the invention. Further, the method of the invention uses at least two different GSH sites. It will be understood that further GSH sites could be used to introduce further transcriptional regulators, such as a third GSH site into which one or more gRNA sequences are inserted.

Insertions specifically within genomic safe harbour sites are preferred over random genome integration, since this is expected to be a safer modification of the genome, and is less likely to lead to unwanted side effects such as silencing of natural gene expression or causing mutations that lead to cancerous cell types.

A genomic safe harbour (GSH) site is a locus within the genome wherein a gene or other genetic material may be inserted without any deleterious effects on the cell or on the inserted genetic material. Most beneficial is a GSH site in which expression of the inserted gene sequence is not perturbed by any read-through expression from neighbouring genes and expression of the inducible cassette minimizes interference with the endogenous transcription programme. More formal criteria have been proposed that assist in the determination of whether a particular locus is a GSH site in future (Papapetrou et al. (2011) Nature Biotechnology, 29(1):73-8, doi: 10.1038/nbt.1717). These criteria include a site that is (i) 50 kb or more from the 5' end of any gene, (ii) 300 kb or more from any gene related to cancer, (iii) 300 kb or more from any microRNA (miRNA), (iv) located outside a transcription unit and (v) located outside ultra-conserved regions (UCR). It may not be necessary to satisfy all of these proposed criteria, since GSH sites already identified do not fulfil all of the criteria. It is thought that a suitable GSH site will satisfy at least 2, 3, 4 or all of these criteria.

Further sites may be identified by looking for sites where viruses naturally integrate without disrupting native gene expression. They may also be defined as sites that cannot be silenced and thus warrant stable transgene expression, such as described in WO2021152086A1 and which are hereby incorporated by reference.

Any suitable GSH site may be used in the method of the invention, on the basis that the site allows insertion of genetic material without deleterious effects to the cell and permits transcription of the inserted genetic material. Those skilled in the art may use this simplified criterion to identify a suitable GSH site, and/or the more formal criteria set out above.

For the human genome, several GSHs have been identified, and these include the AAVS1 locus, the hROSA26 locus and the CLYBL gene. The CCR5 gene has also been mooted as a possible GSH, and further investigation may identify one or more of these as GSHs in the human genome. Additional GSHs have recently been discovered and validated by Aznauryan et al. (Cell Rep Methods, 2022; 2(1):100154), https://doi.org/10.1016/ j.crmeth.2021.100154) which are hereby incorporated by reference.

The adeno-associated virus integration site 1 locus (AAVS1) is located within the protein phosphatase 1, regulatory subunit 12C (PPP1R12C) gene on human chromosome 19, which is expressed uniformly and ubiquitously in human tissues. This site serves as a specific integration locus for AAV serotype 2, and thus was identified as a possible GSH. AAVS1 has been shown to be a favourable environment for transcription, since it comprises an open chromatin structure and native chromosomal insulators that enable resistance of the inducible cassettes against silencing. There are no known adverse effects on the cell resulting from disruption of the PPP1R12C gene. Moreover, an inducible cassette inserted into this site remains transcriptionally active in many diverse cell types. AAVS1 is thus considered to be a GSH and has been widely utilized for targeted transgenesis in the human genome.

The hROSA26 site has been identified on the basis of sequence analogy with a GSH from mice (ROSA26 - reverse oriented splice acceptor site #26). Although the orthologue site has been identified in humans, this site is not commonly used for inducible cassette insertion. The present inventors have developed a targeting system specifically for the hROSA26 site and thus were able to insert genetic material into this locus. The hROSA26 locus is on chromosome 3 (3p25.3), and can be found within the Ensembl database (GenBank: CR624523). The integration site lies within the open reading frame (ORF) of the *THUMPD3* long non-coding RNA (reverse strand). Since the hROSA26 site has an endogenous promoter, the inserted genetic material may take advantage of that endogenous promoter, or alternatively may be inserted operably linked to an exogenous promoter.

Intron 2 of the Citrate Lyase Beta-like (CLYBL) gene, on the long arm of Chromosome 13, was identified as a suitable GSH since it is one of the identified integration hot-spots of the phage derived phiC31 integrase. Studies have demonstrated that randomly inserted inducible cassettes into this locus are stable and expressed. It has been shown that insertion of inducible cassettes at this GSH do not perturb local gene expression (Cerbibi et al, 2015, PLOS One, DOI:10.1371). CLYBL thus provides a GSH which may be suitable for use in the present invention.

CCR5, which is located on chromosome 3 (position 3p21.31) is a gene which codes for HIV-1 major co-receptor. Interest in the use of this site as a GSH arises from the null mutation in this gene that appears to have no adverse effects, but predisposes to HIV-1 infection resistance. Zinc-finger nucleases that target the third exon have been developed, thus allowing for insertion of genetic material at this locus. Given that the natural function of CCR5 has yet to be elucidated, the site remains a putative GSH which may have utility for the present invention.

GSHs in other organisms have been identified and include ROSA26, HPRT and Hipp11 (H11) loci in mice. Mammalian genomes may include GSH sites based upon pseudo attP sites. For such sites, hiC31 integrase, the *Streptomyces* phage-derived recombinase, has been developed as a non-viral insertion tool, because it has the ability to integrate an inducible cassette-containing plasmid carrying an attB site into pseudo attP sites.

GSHs are also present in the genomes of plants, and modification of plant cells can form part of the present invention. GSH sites have been identified in the genomes of rice (Cantos et al. (2014) Front. Plant Sci., 5(302), doi: http://dx.doi.org/10.3389/ fpls.2014.00302).

Thus, in one embodiment the first, second and third GSH sites are selected from any three of: the hROSA26 locus, the AAVS1 locus, the *CLYBL* gene and/or the CCR5 gene, such as wherein the first genomic safe harbour site is the hROSA26 locus, the second genomic safe harbour site is the AAVS1 locus and the third genomic safe harbour site is the *CLYBL* gene. In another embodiment, the first and second GSH sites are selected from any two of: the hROSA26 locus, the AAVS1 locus, the *CLYBL* gene and/or the CCR5 gene, such as wherein the first genomic safe harbour site is the hROSA26 locus and the second genomic safe harbour site is the AAVS1 locus or the *CLYBL* gene.

In the methods of the invention, insertions occur at different GSH sites, thus at least two GSH sites are required for the method of the invention. The first GSH site is modified by insertion of at least a gene encoding a transcriptional regulator protein. The second GSH site is modified by the insertion of an inducible cassette which comprises a genetic sequence operably linked to an inducible promoter. Other genetic material may also be inserted with either or both of these elements. The genetic sequence operably linked to an inducible promoter within the inducible cassette is preferably a DNA sequence that encodes an mRNA encoding for a catalytically inactive programmable nuclease. The transcription is controlled using the inducible promoter.

In one embodiment, insertion of the gene encoding a transcriptional regulator protein into the first GSH site occurs on both chromosomes of the cell. In a further embodiment, insertion of the inducible cassette into the second GSH site occurs on both chromosomes of the cell.

In one embodiment, one or more guide RNA (gRNA) sequences are inserted into the first GSH site. Said one or more gRNA sequences are complementary to one or more transcription start sites (TSSs) of an endogenous lineage-specific factor gene. Thus, in a further aspect of the invention there is provided a method of forward programming an induced pluripotent stem cell (iPSC) into a somatic cell, said method comprising:
(i) targeted insertion of a gene encoding a transcriptional regulator protein and insertion of one or more guide RNA (gRNA) sequences into a first genomic safe harbour (GSH) site, wherein said one or more gRNA sequences are complementary to one or more transcription start sites (TSSs) of an endogenous lineage-specific factor gene and is operably linked to a constitutive promoter; and
(ii) targeted insertion of an inducible cassette into a second GSH site, wherein said inducible cassette comprises a genetic sequence encoding a catalytically inactive programable nuclease protein and one or more transcription activator proteins operably linked to an inducible promoter, and wherein said inducible promoter is regulated by the transcriptional regulator protein;
wherein the first and second GSH sites are different.

In an alternative embodiment, one or more gRNA sequences are inserted into the second GSH site. Thus, in another aspect of the invention, there is provided a method of forward programming an induced pluripotent stem cell (iPSC) into a somatic cell, said method comprising:
(i) targeted insertion of a gene encoding a transcriptional regulator protein into a first genomic safe harbour (GSH) site; and
(ii) targeted insertion of an inducible cassette into a second GSH site, wherein said inducible cassette comprises one or more guide RNA (gRNA) sequences, a genetic sequence encoding a catalytically inactive programable nuclease protein and one or more transcription activator proteins operably linked to an inducible promoter, wherein said one or more gRNA sequences are complementary to one or more transcription start sites (TSSs) of an endogenous lineage-specific factor gene, and wherein said inducible promoter is regulated by the transcriptional regulator protein;
wherein the first and second GSH sites are different.

In a further alternative embodiment, the one or more gRNA sequences are inserted into the third GSH site, wherein said third GSH site is different to the first and second GSH sites. Thus, in certain embodiments insertion occurs at three different GSH sites. In a further embodiment, insertion of the one or more gRNA sequence into the third GSH site occurs on both chromosomes of the cell.

The first GSH site can be any suitable GSH site and the third GSH site can be any other suitable GSH site. Optionally, the first and third GSH sites are GSHs with an endogenous promoter that is constitutively expressed; which will result in the inserted transcriptional regulator protein and/or gRNA being constitutively expressed. A suitable GSH is the hROSA26 site for human cells. Alternatively, the inserted transcriptional regulator protein and/or gRNA is operably linked to a promoter, preferably a constitutive promoter. A constitutive promoter can be used in conjunction with an insertion in the hROSA26 site. Thus, in one embodiment the transcriptional regulator protein and/or the gRNA sequence are constitutively expressed.

### Transcriptional Regulator Proteins & Inducible Transcription

A transcriptional regulator protein is a protein that binds to DNA, preferably sequencespecifically to a DNA site located in or near a promoter, and either facilitates the binding of the transcription machinery to the promoter, and thus transcription of the DNA sequence (e.g., a transcriptional activator) or blocks this process (e.g., a transcriptional repressor). Such entities are also known as transcription factors.

The DNA sequence that a transcriptional regulator protein binds to is called a transcription factor-binding site or response element, and these are found in or near the promoter of the regulated DNA sequence.

Transcriptional activator proteins bind to a response element and promote gene expression. Such proteins are preferred in the methods of the present invention for controlling inducible cassette expression.

Transcriptional repressor proteins bind to a response element and prevent gene expression. Transcriptional regulator proteins may be activated or deactivated by a number of mechanisms including binding of a substance, interaction with other transcription factors (e.g., homo- or hetero-dimerization) or coregulatory proteins, phosphorylation, methylation and/or by light. The transcriptional repressor may be controlled by activation or deactivation.

If the transcriptional regulator protein is a transcriptional activator protein, it is preferred that the transcriptional activator protein requires activation. This activation may be through any suitable means, but it is preferred that the transcriptional regulator protein is activated through the addition to the cell of an exogenous substance. The supply of an exogenous substance to the cell can be controlled, and thus the activation of the transcriptional regulator protein can be controlled. Alternatively, an exogenous substance can be supplied in order to deactivate a transcriptional regulator protein, and then withdrawn in order to activate the transcriptional regulator protein. Thus, in one embodiment the activity of the transcriptional regulator protein is controlled by an exogenous substance.

If the transcriptional regulator protein is a transcriptional repressor protein, it is preferred that the transcriptional repressor protein requires deactivation. Thus, a substance is supplied to prevent the transcriptional repressor protein repressing transcription, and thus transcription is permitted.

Any suitable transcriptional regulator protein may be used, preferably one that is activatable or deactivatable. It is preferred that an exogenous substance may be supplied to control the transcriptional regulator protein. Such transcriptional regulator proteins are also called inducible transcriptional regulator proteins.

Tetracycline-Controlled Transcriptional Activation is a method of inducible gene expression where transcription is reversibly turned on or off in the presence of the antibiotic tetracycline or one of its derivatives (*e.g.*, doxycycline (which is more stable) or minocycline). In this system, the transcriptional activator protein is tetracycline-responsive transcriptional activator protein (rtTa) or a derivative thereof. The rtTA protein is able to bind to DNA at specific tet operator (TetO) sequences. Several repeats of such TetO sequences are placed upstream of a minimal promoter (such as the CMV promoter), which together form a tetracycline response element (TRE). There are two forms of this system, depending on whether the addition of tetracycline or a derivative activates (Tet-On) or deactivates (Tet-Off) the rtTA protein.

In a Tet-Off system, tetracycline or a derivative thereof binds rtTA and deactivates the rtTA, rendering it incapable of binding to TRE sequences, thereby preventing transcription of TRE-controlled genes. This system was first described in Bujard, et al (1992). Proc. Natl. Acad. Sci. U.S.A. 89 (12): 5547-51.

The Tet-On system is composed of two components; (1) the constitutively expressed tetracycline-responsive transcriptional activator protein (rtTa) and the rtTa sensitive inducible promoter (Tet Responsive Element, TRE). rtTA binds to the TRE-containing promoter in the presence of tetracycline or its more stable derivatives, including doxycycline (dox), resulting in activation of rtTa, inducing expression of TRE-controlled genes. The use of this may be preferred in the method of the invention.

Thus, the transcriptional regulator protein may be tetracycline-responsive transcriptional activator protein (rtTa) protein, which can be activated or deactivated by the antibiotic tetracycline or one of its derivatives, which are supplied exogenously. If the transcriptional regulator protein is rtTA, then the inducible promoter inserted into the second GSH site includes the tetracycline response element (TRE). The exogenously supplied substance is the antibiotic tetracycline or one of its derivatives.

Variants and modified rtTa proteins may be used in the methods of the invention, these include Tet-On Advanced transactivator (also known as rtTA2S-M2) and Tet-On 3G (also known as rtTA-V16, derived from rtTA2S-S2).

The tetracycline response element (TRE) generally consists of 7 repeats of the 19bp bacterial TetO sequence separated by spacer sequences, together with a minimal promoter. Variants and modifications of the TRE sequence are possible, since the minimal promoter can be any suitable promoter. Preferably the minimal promoter shows no or minimal expression levels in the absence of rtTa binding. The inducible promoter inserted into the second GSH site may thus comprise a TRE.

A modified system based upon tetracycline control is the T-REx^{™} System (Thermofisher Scientific), in which the transcriptional regulator protein is a transcriptional repressor protein, TetR. The components of this system include (i) an inducible promoter comprising a strong human cytomegalovirus immediate-early (CMV) promoter and two tetracycline operator 2 (TetO2) sites, and a Tet repressor (TetR). The TetO2 sequences consist of 2 copies of the 19bp sequence, 5'-TCCCTATCAGTGATAGAGA-3' (SEQ ID NO: 25) separated by a 2bp spacer. In the absence of tetracycline, the Tet repressor forms a homodimer that binds with extremely high affinity to each TetO2 sequence in the inducible promoter, and prevents transcription from the promoter. Once added, tetracycline binds with high affinity to each Tet repressor homodimer rendering it unable to bind to the Tet operator. The Tet repressor:tetracycline complex then dissociates from the Tet operator and allows induction of expression. In this instance, the transcriptional regulator protein is TetR and the inducible promoter comprises two TetO2 sites. The exogenously supplied substance is tetracycline or a derivative thereof.

The cumate switch is another method of inducible gene expression where transcription is reversibly turned on or off in the presence of the cumate. This system is available in both activator and repressor configurations, where the presence of cumate leads to the repression of transcription or activation of transcription, respectively. In the repressor configuration, regulation is mediated by the binding of the repressor (CymR) to the operator site (CuO), placed downstream of a constitutive promoter. Addition of cumate, a small molecule, relieves the repression and allows transcription to proceed. In the activator configuration, a chimeric transactivator (cTA) protein, formed by the fusion of CymR with the activation domain of VP16, is able to activate transcription when bound to the CuO operator site, placed upstream of the constitutive promoter. Cumate addition abrogates DNA binding and therefore transactivation by cTA, stopping transcription.

Other inducible expression systems are known and can be used in the method of the invention. These include the Complete Control Inducible system from Agilent Technologies. This is based upon the insect hormone ecdysone or its analogue ponasterone A (ponA) which can activate transcription in mammalian cells which are transfected with both the gene for the *Drosophila melongaster* ecdysone receptor (EcR) and an inducible promoter comprising a binding site for the ecdysone receptor. The EcR is a member of the retinoid-X-receptor (RXR) family of nuclear receptors. In humans, EcR forms a heterodimer with RXR that binds to the ecdysone-responsive element (EcRE). In the absence of PonA, transcription is repressed by the heterodimer.

Thus, the transcriptional regulator protein can be a repressor protein, such as an ecdysone receptor or a derivative thereof. Examples of the latter include the VgEcR synthetic receptor from Agilent technologies which is a fusion of EcR, the DNA binding domain of the glutocorticoid receptor and the transcriptional activation domain of Herpes Simplex Virus VP16. The inducible promoter comprises the EcRE sequence or modified versions thereof together with a minimal promoter. Modified versions include the E/GRE recognition sequence of Agilent Technologies, in which mutations to the sequence have been made. The E/GRE recognition sequence comprises inverted half-site recognition elements for the retinoid-X-receptor (RXR) and GR binding domains. In all permutations, the exogenously supplied substance is ponasterone A, which removes the repressive effect of EcR or derivatives thereof on the inducible promoter, and allows transcription to take place.

Alternatively, inducible systems may be based on the synthetic steroid mifepristone as the exogenously supplied substance. In this scenario, a hybrid transcriptional regulator protein is inserted, which is based upon a DNA binding domain from the yeast GAL4 protein, a truncated ligand binding domain (LBD) from the human progesterone receptor and an activation domain (AD) from the human NF-κB. This hybrid transcriptional regulator protein is available from Thermofisher Scientific under the trade name Gene Switch^{™}. Mifepristone activates the hybrid protein, and permits transcription from the inducible promoter which comprises GAL4 upstream activating sequences (UAS) and the adenovirus E1b TATA box. This system is described in Wang, Y. et al (1994) Proc. Natl. Acad. Sci. USA 91, 8180-8184.

The transcriptional regulator protein can thus be any suitable regulator protein, either an activator or repressor protein. Suitable transcriptional activator proteins are tetracycline-responsive transcriptional activator protein (rtTa) or the Gene Switch^{™} hybrid transcriptional regulator protein. Suitable repressor proteins include the Tet-Off version of rtTA, TetR or EcR. The transcriptional regulator proteins may be modified or derivatised as required.

Thus, in one embodiment the transcriptional regulator protein is selected from any one of: a tetracycline-responsive transcriptional activator protein (rtTa), a tetracycline repressor (TetR), VgEcR synthetic receptor, a cumate repressor (CymR) or a hybrid transcriptional regulator protein comprising a DNA binding domain from the yeast GAL4 protein, a truncated ligand binding domain from the human progesterone receptor and an activation domain from the human NF-κB. In a particular embodiment the transcriptional regulator protein is rtTA and its activity is controlled by tetracycline of a derivative thereof, such as doxycycline. According to this particular embodiment, the inducible promoter may include a tetracycline-responsive element (TRE).

In one embodiment, the transcription of sequences inserted into the first GSH site is under the control of one or more constitutive promoters and the transcription of sequences inserted into the second GSH site is under the control of an inducible promoter. In another embodiment, the transcription of sequences inserted into the first and third GSH sites is under the control of one or more constitutive promoters and the transcription of sequences inserted into the second GSH site is under the control of an inducible promoter.

The inducible promoter can comprise elements which are suitable for binding or interacting with the transcriptional regulator protein. The interaction of the transcriptional regulator protein with the inducible promoter is preferably controlled by the exogenously supplied substance.

The exogenously supplied substance can be any suitable substance that binds to or interacts with the transcriptional regulator protein. Suitable substances include tetracycline, tetracycline derivatives (e.g., doxycycline), ponasterone A and mifepristone.

Thus, the insertion of the gene encoding a transcriptional regulator protein into the first GSH site provides the control mechanism for the expression of the inducible cassette which is operably linked to the inducible promoter and inserted into a second, different, GSH site.

The transcriptional regulator protein gene may be provided for insertion with other genetic material. Such material includes genes for markers or reporter molecules, such as genes that induce visually identifiable characteristics including fluorescent and luminescent proteins. Examples include the gene that encodes jellyfish green fluorescent protein (GFP), which causes cells that express it to glow green under blue/UV light, luciferase, which catalyses a reaction with luciferin to produce light, and the red fluorescent protein from the gene dsRed. Such markers or reporter genes are useful, since the presence of the reporter protein confirms protein expression from the first GSH site, indicating successful insertion. Selectable markers may further include resistance genes to antibiotics or other drugs. Non-inducible expression of molecular tools may also be desirable, including optogenetic tools, nuclear receptor fusion proteins, such as tamoxifen-inducible systems ERT, and designer receptors exclusively activated by designer drugs. Furthermore, sequences that code signalling factors that alter the function of the same cell or of neighbouring or even distant cells in an organism, including hormones, autocrine or paracrine factors may be co-expressed from the same GSH site as the transcriptional regulator protein.

Additionally, the further genetic material may include sequences coding for non-coding RNA, as discussed herein. Examples of such genetic material include gRNA sequences and genes for miRNA, which may function as a genetic switch.

It is preferred that the gene encoding the transcriptional regulator protein is operably linked to a constitutive promoter. Alternatively, the first GSH site can be selected such that it already has a constitutive promoter that can also drive expression of the transcriptional regulator protein gene and any associated genetic material. Constitutive promoters ensure sustained and high level gene expression. Commonly used constitutive promoters include the human β-actin promoter (ACTB), cytomegalovirus (CMV), elongation factor-1α, (EF1α), phosphoglycerate kinase (PGK) and ubiquitinC (UbC). The CAG promoter is a strong synthetic RNA Polymerase II promoter frequently used to drive high levels of gene expression and was constructed from the following sequences: (C) the cytomegalovirus (CMV) early enhancer element, (A) the promoter, the first exon and the first intron of chicken beta-actin gene, and (G) the splice acceptor of the rabbit beta-globin gene. Further examples of suitable constitutive promoters include RNA Polymerase III promoters, such as the human U6 promoter, mouse U6 promoter or human H1 promoter.

Further, the transcriptional regulator, plus any additional genetic material may be provided together with cleavable sequences. Such sequences are sequences that are recognised by an entity capable of specifically cutting DNA, and include restriction sites, which are the target sequences for restriction enzymes or sequences for recognition by other DNA cleaving entities, such as nucleases, recombinases, ribozymes or artificial constructs. At least one cleavable sequence may be included, but preferably two or more are present. These cleavable sequences may be at any suitable point in the insertion, such that a selected portion of the insertion, or all of the insertion, can be selectively removed from the GSH site. The method can thus extend to removal and/or replacement of the insertion or a portion thereof from the GSH. The cleavable sites may thus flank the part/all of the insertion that it may be desired to remove. The transcriptional regulator and/or the further genetic material may be removed using this method.

A portion of the insertion may be any part up to 99% of the insertion - i.e. 1-99%, 90%, 80%, 70%, 60%, 50%, 40%, 30%, 20%, 10% or less than 10%.

It may be preferred that the portion of the insertion flanked by the cleavable sites includes the constitutive promoter. Alternatively, the constitutive promoter is not included in the portion flanked by the cleavable sequences.

A preferred cleavable sequence is the IoxP site for Cre recombinase as it allows direct replacement of the removed insertion. Alternatively or additionally, the cleavable sequence is the rox site for Dre recombinase.

It is preferred that the insertion at the first GSH site occurs at both loci in the genome, thus each allele is modified by insertion. This permits greater expression from the gene encoding the transcriptional regulator and any associated genetic material.

The second GSH site can be any suitable GSH site. It may be preferred that the second GSH site is not associated with an endogenous promoter, such that the expression of the inserted inducible cassette is solely under control of the transcriptional regulator protein.

### Inducible Cassettes

An inducible cassette includes a desired genetic sequence, preferably a DNA sequence that is to be transferred into a cell. In methods of the invention, the introduction of an inducible cassette into the genome has the potential to change the phenotype of that cell through activation of an endogenous gene. In particular, the inducible cassette comprises a genetic sequence encoding a catalytically inactive programable nuclease protein and one or more transcription activator proteins, wherein said genetic sequence is operably linked to an inducible promoter. Once expressed, the catalytically inactive programmable nuclease or a fusion protein comprising the catalytically inactive programmable nuclease is targeted by one or more gRNA sequences complementary to one or more transcription start sites of an endogenous lineage-specific factor gene, thereby resulting in expression of the endogenous lineage-specific factor gene. In some embodiments, the inducible cassette further comprises a sequence encoding a nuclease (*e.g*., an endonuclease, such as Csy4) which recognises and cleaves the cleavable sequences comprised in a multiple gRNA sequence array as described herein. Said nuclease, once expressed, processes the array to cleave and separate the gRNA sequences, allowing them to be recruited by the catalytically inactive programmable nuclease or a fusion protein thereof and the resulting targeting to multiple (*e.g*., to two or more) TSSs of an endogenous lineage-specific factor gene or to multiple endogenous lineage-specific factor gene TSSs.

The inducible cassette includes a genetic sequence operably linked to an inducible promoter. A "promoter" is a nucleotide sequence which initiates and regulates transcription of a polynucleotide. An "inducible promoter" is a nucleotide sequence where expression of a genetic sequence operably linked to the promoter is controlled by an analyte, co-factor, regulatory protein, etc. In the case of the present invention, the control is effected by the transcriptional regulator protein. It is intended that the term "promoter" or "control element" includes full-length promoter regions and functional (*e.g*., controls transcription or translation) segments of these regions. "Operably linked" refers to an arrangement of elements wherein the components so described are configured so as to perform their usual function. Thus, a given promoter operably linked to a genetic sequence is capable of effecting the expression of that sequence when the proper enzymes are present. The promoter need not be contiguous with the sequence, so long as it functions to direct the expression thereof. Thus, for example, intervening untranslated yet transcribed sequences can be present between the promoter sequence and the genetic sequence and the promoter sequence can still be considered "operably linked" to the genetic sequence. Thus, the term "operably linked" is intended to encompass any spacing or orientation of the promoter element and the genetic sequence in the inducible cassette which allows for initiation of transcription of the inducible cassette upon recognition of the promoter element by a transcription complex.

The transcriptional regulator protein and the inducible cassette, together with any associated genetic material, are inserted into different GSH sites within the genome of the cell.

### Site-Specific Delivery & Targeting

Preferably, the insertions into the GSH sites are specifically within the sequence of the GSH as described previously. Any suitable technique for insertion of a polynucleotide into a specific sequence may be used, and several are described in the art. Suitable techniques include any method which introduces a break at the desired location and permits recombination of a donor vector into the gap. Thus, a crucial first step for targeted site-specific genomic modification is the creation of a double-strand DNA break (DSB) at the genomic locus to be modified. Distinct cellular repair mechanisms can be exploited to repair the DSB and to introduce the desired sequence, and these are non-homologous end joining repair (NHEJ), which is more prone to error; and homologous recombination repair (HR) mediated by a donor DNA template, that can be used to insert inducible cassettes. In some embodiments, the insertions into the GSH sites are performed using the techniques disclosed in WO 2018/096343, which are hereby incorporated by reference. Thus, any method of making specific, targeted double strand breaks in the genome in order to insert a gene/inducible cassette may be used in the method of the invention. It may be preferred that the method for inserting the gene/inducible cassette utilises any one or more of ZFNs, TALENs and/or CRISPR/Cas9 systems or any derivative thereof.

Once the DSB has been made by any appropriate means, the gene/inducible cassette for insertion may be supplied in any suitable fashion as described below. The gene/inducible cassette and associated genetic material form the donor DNA for repair of the DNA at the DSB and are inserted using standard cellular repair machinery/pathways. How the break is initiated will alter which pathway is used to repair the damage, as noted above.

The transcriptional regulator protein and the inducible cassette may be supplied for the method of the invention on separate vectors. A "vector" is a nucleic acid molecule, such as a DNA molecule, which is used as a vehicle to artificially transfer exogenous genetic material into a cell. The vector is generally a nucleic acid sequence that consists of an insert (such as an inducible cassette or gene for a transcriptional regulator protein) and a larger sequence that serves as the "backbone" of the vector. The vector may be in any suitable format, including plasmids, minicircle, linear DNA or a single-stranded AAV template. The vector comprises at least the gene for the transcriptional regulator or inducible cassette operably linked to an inducible promoter, together with the minimum sequences to enable insertion of the genes into the relevant GSH. Optionally, the vectors also possess an origin of replication (ori) which permits amplification of the vector, for example in bacteria. Additionally or alternatively, the vector includes selectable markers such as antibiotic resistance genes, genes for coloured markers and/or suicide genes.

Vectors include but are not limited to plasmids, cosmids, viruses (bacteriophage, animal viruses and plant viruses) and artificial chromosomes (*e.g.*, yeast artificial chromosomes or YACs).

In one embodiment, the vector(s) is a viral vector. The viral gene delivery system may be an RNA-based or DNA-based viral vector. Viral vectors include retroviral vectors (*e.g*., lentiviral vectors, such as those derived from HIV-1, HIV-2, SIV, BIV, FIV etc.), gammaretroviral vectors, adenoviral (Ad) vectors (including replication competent, replication deficient and gutless forms thereof), adeno-associated virus-derived (AAV) vectors, simian virus 40 (SV-40) vectors, bovine papilloma virus vectors, Epstein-Barr virus vectors, herpes virus vectors, vaccinia virus vectors, Harvey murine sarcoma virus vectors, murine mammary tumour virus vectors, Rous sarcoma virus vectors and Sendai virus vectors. In a further embodiment, the viral vector(s) is selected from: a lentiviral vector, an adeno-associated virus vector or a Sendai virus vector. In a yet further embodiment, the viral vector(s) is a lentiviral vector.

Lentiviral vectors are well known in the art. Lentiviral vectors are complex retroviruses capable of integrating randomly into the host cell genome, which, in addition to the common retroviral genes gag, pol, and env, contain other genes with regulatory or structural function (*e.g*., accessory genes Vif, Nef, Vpu, Vpr). Lentiviral vectors have the advantage of being able to infect non-dividing cells and can be used for both *in vivo* and *ex vivo* gene transfer and expression of nucleic acid sequences. For example, a recombinant lentiviral vector is capable of infecting a non-dividing cell wherein a suitable host cell is transfected with two or more vectors carrying the packaging functions, namely gag, pol and env, as well as rev and tat.

Thus, in some embodiments the targeted insertion at the GSH site is obtained in the absence of a DNA double-strand break. This may be achieved using adeno-associated virus (AAV) for targeting. In this instance, AAV is modified to contain the transgene, alongside with a suitable selection marker (PuroR, BlaR, HygroR, ZeoR or eGFP, mCherry, DsRed) flanked by suitable homology arms. Infection of the target cells may produce a genetically engineered cell in which the target locus (GSH site) contains the transgene.

In another embodiment, the vector(s) is a plasmid. The plasmid(s) may be episomal. Episomal vectors are able to introduce large fragments of DNA into a cell but are maintained extra-chromosomally, replicated once per cell cycle, partitioned to daughter cells efficiently, and elicit substantially no immune response. Alternatively, an Epstein-Barr virus (EBV)-based episomal vector, a yeast-based vector, an adenovirus-based vector, a simian virus 40 (SV40)-based episomal vector, or a bovine papilloma virus (BPV)-based vector may be used.

In particular embodiments, the inducible cassette comprises a genetic sequence that encodes a catalytically inactive programmable nuclease. In further embodiments, the inducible cassette comprises a genetic sequence that encodes one or more transcription activator proteins. Thus, in one embodiment the inducible cassette comprises a genetic sequence encoding a catalytically inactive programmable nuclease and one or more transcription activator proteins. In a further embodiment, the genetic sequence encoding the catalytically inactive programmable nuclease is contiguous with the sequence encoding one or more transcription activator proteins. In a yet further embodiment the one or more transcription activator proteins is fused to the catalytically inactive programmable nuclease, i.e. the catalytically inactive programmable nuclease and the one or more transcription activator proteins are a fusion protein.

Thus, according to a further aspect of the invention, there is provided a method of forward programming an induced pluripotent stem cell (iPSC) into a somatic cell, said method comprising:
(i) targeted insertion of a gene encoding a transcriptional regulator protein into a first genomic safe harbour (GSH) site;
(ii) targeted insertion of an inducible cassette into a second GSH site, wherein said inducible cassette comprises a genetic sequence encoding a catalytically inactive programable nuclease protein fused to one or more transcription activator proteins operably linked to an inducible promoter, and wherein said inducible promoter is regulated by the transcriptional regulator protein; and
(iii) targeted insertion of one or more guide RNA (gRNA) sequences into a third GSH site, wherein said one or more gRNA sequence is complementary to one or more transcription start sites (TSSs) of an endogenous lineage-specific factor gene and is operably linked to a constitutive promoter,
wherein the first, second and third GSH sites are different.

In a further aspect, there is provided a method of forward programming an induced pluripotent stem cell (iPSC) into a somatic cell, said method comprising:
(i) targeted insertion of a gene encoding a transcriptional regulator protein and insertion of one or more guide RNA (gRNA) sequences into a first genomic safe harbour (GSH) site, wherein said one or more gRNA sequence is complementary to one or more transcription start sites (TSSs) of an endogenous lineage-specific factor gene and is operably linked to a constitutive promoter; and
(ii) targeted insertion of an inducible cassette into a second GSH site, wherein said inducible cassette comprises a genetic sequence encoding a catalytically inactive programable nuclease protein fused to one or more transcription activator proteins operably linked to an inducible promoter, and wherein said inducible promoter is regulated by the transcriptional regulator protein;
wherein the first and second GSH sites are different.

In another aspect, there is provided a method of forward programming an induced pluripotent stem cell (iPSC) into a somatic cell, said method comprising:
(i) targeted insertion of a gene encoding a transcriptional regulator protein into a first genomic safe harbour site; and
(ii) targeted insertion of an inducible cassette and one or more guide RNA (gRNA) sequences into a second genomic safe harbour site, wherein said inducible cassette comprises, a genetic sequence encoding a catalytically inactive programable nuclease protein fused to one or more transcription activator proteins operably linked to an inducible promoter, wherein said one or more gRNA sequence is complementary to one or more transcription start sites (TSSs) of an endogenous lineage-specific factor gene, and wherein said inducible promoter is regulated by the transcriptional regulator protein;
wherein the first and second genomic safe harbour sites are different.

### Transcription Activator Proteins

Transcription activator proteins as referred to herein promote or activate transcription at the transcription start site (TSS) of an endogenous gene, similar to previously described herein for transcriptional regulator proteins which activate transcription at response elements. Such transcription activator proteins encoded by the genetic sequence comprised in the inducible cassette include: VP16, VP64, p65, Rta, MS2 and HSF1, or fusion proteins thereof. In one embodiment, the one or more transcription activator proteins is selected from one or more of: VP16, VP64, p65, Rta, MS2 and HSF1. In a particular embodiment, the one or more transcription activator protein is VP64. In a further embodiment, the one or more transcription activator proteins is a combination of VP64, p65 and Rta or a combination of MS2, p65 and HSF1. In a yet further embodiment, the transcription activator protein is a VP64-p65-Rta fusion protein. In another embodiment, the transcription activator protein is a MS2-p65-HSF1 fusion protein.

Fusion of the one or more transcription activator proteins (*e.g*., one or more of: VP16, VP64, p65, Rta, MS2 and HSF1, or a fusion protein thereof) to the catalytically inactive programmable nuclease targets the transcription activator proteins to the TSS of an endogenous gene (*e.g.*, an endogenous lineage-specific factor gene) to activate or increase transcription at said TSS. Targeting of the catalytically inactive programmable nuclease to the TSS is in turn driven by the one or more gRNA sequences (*e.g*., inserted into the first, second or third GSH site) through the complementarity of the gRNA sequences to the TSS. In another embodiment, the one or more transcription activator proteins (*e.g*., one or more of: VP16, VP64, p65, Rta, MS2 and HSF1, or a fusion protein thereof) are targeted to the TSS of an endogenous gene by the gRNA sequence itself, *i.e.,* the one or more transcription activator proteins, or at least one of said transcription activator proteins, is not fused to the catalytically inactive programmable nuclease. According to this embodiment, the one or more gRNA sequences may comprise an aptamer sequence to which one or more of the transcription activator proteins bind, such as an MS2 aptamer sequence to which the MS2 transcription activator protein binds. Thus, in a further embodiment the one or more gRNA sequences comprise an MS2 aptamer sequence. In a yet further embodiment, the MS2 aptamer sequence optionally recruits an MS2-containing fusion protein to the TSS of the endogenous lineage-specific factor gene. In some embodiments, the MS2-containing fusion protein is an MS2-p65-HSF1 fusion protein.

### Guide RNA (gRNA) Sequences

Thus, in certain embodiments the one or more gRNA sequences targets the catalytically inactive programmable nuclease and one or more transcription activator proteins to the TSS of the endogenous gene, such as an endogenous lineage-specific factor gene. For example, the one or more gRNA sequences may target the transcription activator proteins to the TSS directly, *e.g.*, through binding of at least one of the transcription activator proteins to an aptamer sequence in the gRNA, or the one or more gRNA sequences may target the transcription activator proteins to the TSS through targeting of the catalytically inactive programmable nuclease to the TSS, *e.g*., because at least one of the transcription activator proteins are fused to the catalytically inactive programmable nuclease. In a further embodiment, the one or more transcription activator proteins activates or increases transcription at an endogenous TSS, such as at the TSS of an endogenous lineage-specific factor gene.

Thus, in certain embodiments the one or more gRNA sequences are complementary to one or more TSSs of an endogenous lineage-specific factor gene. In a particular embodiment, the spacer region(s) of the one or more gRNA sequences are complementary to the TSS of an endogenous lineage-specific factor gene. In some embodiments, the one or more gRNA sequences are single guide RNA (sgRNA) sequences. In further embodiments, the one or more gRNA sequences comprise a trans-activating CRISPR RNA (tracrRNA/tcRNA) region to which the catalytically inactive programmable nuclease binds and a CRISPR RNA (crRNA) region which is complementary to one or more TSS of an endogenous lineage-specific factor gene. Data using insertion of a single gRNA sequence to target a TSS of an endogenous lineage-specific factor gene is provided in Example 1 herein. In one embodiment, two or more gRNA sequences are inserted into the GSH site (*e.g*., the third GSH site). According to this embodiment, each of the gRNA sequences are complementary to alternative or different TSSs of the endogenous lineage-specific factor gene. Alternatively, each of the gRNA sequences are complementary to more than one alternative sequence of the TSS of the endogenous lineage-specific factor gene. Thus, in one embodiment a pool of gRNA sequences is inserted into the GSH site (*e.g.*, the third GSH site). In a particular embodiment, multiple gRNA sequences are inserted into the GSH site (*e.g*., the third GSH site). The insertion of two or more, or a pool of gRNA sequences into the GSH site finds particular utility if the endogenous lineage-specific factor gene has alternative TSSs and/or more than one TSS, such as two or more TSSs, by providing complementary gRNA sequences for each of the alternative and/or multiple TSSs and targeting the catalytically inactive programmable nuclease and/or the one or more transcription activator proteins thereto.

According to embodiments where multiple (*e.g*., two or more), or a pool of gRNA sequences are inserted into the GSH site, said multiple gRNA sequences may be separated by cleavable sequences as described hereinbefore. Such cleavable sequences may be recognised by nucleases, recombinases, ribozymes or artificial constructs (*e.g*., a bacterial DNA endonuclease, such as Csy4). In one embodiment, the multiple gRNA sequences are provided in an array comprising a promoter, two or more gRNA sequences and two or more cleavable sequences. In a particular embodiment, the multiple gRNA sequences are provided in an array comprising a constitutive promoter (*e.g.*, the hU6 promoter), two gRNA sequences and two cleavable sequences. In a further embodiment, each guide RNA sequence may be driven by a separate promoter, *e.g.*, a first gRNA driven by the human U6 promoter, a second gRNA driven by the mouse U6 promoter, a third gRNA driven by the bovine U6 promoter, a fourth gRNA driven by the H1 promoter and so on. Data showing the beneficial impact of multiple guide RNAs targeting the same TSS are provided in Example 2 herein.

Where a multiple gRNA sequence array comprising cleavable sequences is provided, a sequence encoding a nuclease which recognises and cleaves the cleavable sequences may be inserted into any GSH site described herein. In particular, the nuclease is an endonuclease which recognises and cleaves the cleavable sequences of the multiple gRNA sequence array and is encoded by a sequence inserted into the second GSH site with the catalytically inactive programmable nuclease and the one or more transcriptional activator proteins. Thus, in one embodiment a sequence encoding the endonuclease which recognises and cleaves the cleavable sequences of the multiple gRNA sequence array is comprised in the inducible cassette described herein. In an alternative embodiment, a sequence encoding the endonuclease which recognises and cleaves the cleavable sequences of the multiple gRNA sequence array is inserted into the same GSH site as the gRNA sequence array (*e.g*., the first or third GSH site). In another embodiment, a sequence encoding the endonuclease which recognises and cleaves the cleavable sequences of the multiple gRNA sequence array is inserted into a different GSH site to the inducible cassette and the gRNA sequence array. In one embodiment, the endonuclease which recognises and cleaves the cleavable sequences of the multiple gRNA sequence array is expressed as a fusion protein with the programmable nuclease. In another embodiment, the endonuclease which recognises and cleaves the cleavable sequences of the multiple gRNA sequence array is expressed from a multi-cistronic mRNA and linked to the programmable nuclease via P2A, T2A, E2A or an IRES. In yet another embodiment, the endonuclease which recognises and cleaves the cleavable sequences of the multiple gRNA sequence array is delivered separately, e.g., by plasmid transfection, electroporation, lentiviral infection or AAV infection, and thus resides in a locus distinct from the GSH sites mentioned herein.

In one embodiment, the one or more gRNA sequences are operably linked to a constitutive promoter. In one embodiment, the promoter is an RNA Polymerase III-driven promoter, such as human U6, mouse U6, bovine U6 or the human H1 promoter. In a further embodiment, the one or more gRNA sequences inserted into the first GSH site are operably linked to a constitutive promoter, such as an RNA Polymerase III-driven promoter. When inserted into the first GSH site, the one or more gRNA sequences may be operably linked to a different constitutive promoter to the gene encoding a transcriptional regulator protein. In another embodiment, the one or more gRNA sequences inserted into the second GSH site are operably linked to a constitutive promoter, i.e. wherein the gRNA sequences are operably linked to a different promoter to the inducible cassette. In a yet further embodiment, the one or more gRNA sequences inserted into the third GSH site are operably linked to a constitutive promoter.

Therefore, the methods presented herein comprise CRISPRa which uses modified versions of CRISPR effectors which do not have endonuclease activity but comprise added transcriptional activators on the catalytically inactive programmable nuclease (e.g., dCas9 and dCas12a, such as fused to the catalytically inactive programmable nuclease) and/or the gRNAs. The transcriptional activators fused to the CRISPRa components or which bind thereto (e.g., through the presence of aptamer sequences) therefore increase expression of genes of interest following targeting to the gene by the gRNA. Thus, according to some embodiments the programmable nuclease is catalytically inactive. Catalytically inactive programmable nucleases may also be referred to as 'dead'. The programmable nuclease may be rendered catalytically inactive through point mutations in the endonuclease domain(s), such as the RuvC and HNH domains in Cas9. Examples of such point mutations in the endonuclease domains of catalytically inactive Cas9 (also known as dCas9) are D10A and H840A which result in its deactivation. However, as will be readily appreciated such mutations do not affect the ability of the programmable nuclease to bind gRNA and to the targeted gene as such binding is affected by other domains.

### Catalytically Inactive Programmable Nuclease Proteins

In one embodiment, the catalytically inactive programmable nuclease is catalytically inactive Cas9 *(i.e.,* dCas9 as described hereinbefore). In another embodiment, the catalytically inactive programmable nuclease is catalytically inactive Cas12a *(i.e.,* dCas12a). In further embodiments, the catalytically inactive programmable nuclease (*e.g*., dCas9 or dCas12a) comprises point mutations in the RuvCI and HNH nuclease domains. In a particular embodiment, the catalytically inactive programmable nuclease is dCas9 and comprises the point mutations D10A and H840A compared to the wild-type sequence of Cas9.

In alternative embodiments, the catalytically inactive programmable nuclease is a catalytically inactive zinc finger nuclease (ZFN) or a transcription activator-like effector nuclease (TALEN; Gaj, T, et al. (2013) "ZFN, TALEN, and CRISPR/Cas-based methods for genome engineering" Trends Biotechnol, 31:397-405).

Zinc finger nucleases are artificial enzymes which are generated by fusion of a zinc-finger DNA-binding domain to the nuclease domain of the restriction enzyme Fokl. The latter has a non-specific cleavage domain which must dimerise in order to cleave DNA. This means that two ZFN monomers are required to allow dimerisation of the Fokl domains and cleavage of the DNA. The ZFN DNA binding domain comprises a tandem array of Cys₂His₂ zinc fingers, each of which recognises three contiguous nucleotides in the target sequence, and may be designed to target any genomic sequence of interest. The two binding sites are separated by 5-7bp to allow optimal dimerisation of the Fokl domains. The catalytically capable enzyme is thus able to cleave DNA at a specific site. Target specificity is increased by ensuring that two proximal DNA-binding events must occur to achieve a double-strand break. However, as will be appreciated from the disclosures herein, ZFNs to be used as "catalytically inactive nuclease proteins" in the present methods will comprise a catalytically inactive Fokl nuclease domain, or may not comprise a Fokl nuclease domain.

Transcription activator-like effector nucleases, or TALENs, are dimeric transcription factor/nucleases. They are made by fusing a TAL effector DNA-binding domain to a DNA cleavage domain (a deoyribonuclease). Transcription activator-like effectors (TALEs) can be engineered to bind practically any desired DNA sequence, so when combined with a nuclease, DNA can be cut at specific locations. TAL effectors are proteins that are secreted by *Xanthomonas* bacteria, the DNA binding domain of which contains a repeated highly conserved 33-34 amino acid sequence with divergent 12^{th} and 13^{th} amino acids. These two positions are highly variable and show a strong correlation with specific nucleotide recognition. This straightforward relationship between amino acid sequence and DNA recognition has allowed for the engineering of specific DNA-binding domains by selecting a combination of repeat segments containing appropriate residues at the two variable positions. TALENs are thus built from arrays of 33 to 35 amino acid modules, each of which targets a single nucleotide. By selecting the array of the modules, almost any sequence may be targeted. Again, the nuclease used may be Fokl or a derivative thereof, however as will be appreciated, TALENs to be used as "catalytically inactive nuclease proteins" in the present methods will comprise a catalytically inactive Fokl nuclease domain, or may not comprise a Fokl nuclease domain.

### Endogenous Lineage-Specific Factor Genes

As described hereinbefore, the methods of the invention are for forward programming an iPSC into a somatic cell, comprising targeting one or more TSS of an endogenous lineage-specific factor gene. Thus, in one embodiment the lineage-specific factor gene is not expressed from exogenously provided sequence. References herein to "endogenous" therefore refer to the normal meaning of said term, *i.e.,* wherein the lineage-factor gene originates from within the iPSC itself and is not introduced or inserted by methods described hereinbefore.

References herein to "lineage-specific factor gene" refer to genes (*e.g.*, protein coding genes) specifically or necessarily involved in the determination of the identity of a particular cell type, *i.e.,* a lineage. Such factors include lineage-specific transcription factors which are involved in gene regulation in both prokaryotic and eukaryotic organisms. In one embodiment, lineage-specific transcription factors can have a positive effect on gene expression and thus may be referred to as an "activator" or a "transcriptional activation factor". In another embodiment, a lineage-specific transcription factor can negatively affect gene expression and thus may be referred to as "repressors" or a "transcription repression factor". Activators and repressors are generally used terms and their functions may be discerned by those skilled in the art.

The specific identity of endogenous lineage-specific factor genes targeted by the methods described herein will be appreciated to vary depending on the desired lineage of the somatic cell resulting from forward programming of an iPSC. The term "somatic cell" as used herein includes any type *(i.e.,* lineage) of cell that makes up the body of an organism, excluding germ cells and undifferentiated stem cells. Somatic cells may therefore include, for example and without limitation, neurons, glia cells, white blood cells (*e.g.,* leucocytes), liver cells (*e.g.,* hepatocytes), muscle cells (*e.g.*, myocytes) or fibroblasts. In one embodiment, the somatic cell may be an adult cell or a cell derived from an adult which displays one or more detectable characteristics of an adult or non-embryonic cell. References herein to a somatic cell derived from an iPSC by "forward programming" refer to cells which comprise the phenotype and/or characteristics of a somatic cell as defined herein (*e.g.*, the surface phenotype and/or functional characteristics associated with a particular lineage) and have been forward programmed from a pluripotent stem cell which has previously been reprogrammed, *i.e.,* an iPSC. Forward programming of an iPSC to a somatic cell may comprise increasing the expression of endogenous lineage-specific factors (*e.g*., lineage-specific transcription factors), such as by increasing the expression of said lineage-specific factor genes and/or their protein expression. The expression of an endogenous transcription factor may be increased. In one embodiment, forward programming comprises increasing the expression of one or more endogenous lineage-specific factor gene in an iPSC and culturing the cell under conditions suitable for forward programming the cell into a somatic cell.

As described hereinbefore, increasing the expression of the one or more endogenous lineage-specific factor genes in an iPSC may be by CRISPRa. Thus, in some embodiments the expression of the one or more endogenous lineage-specific factor genes is increased by the targeting of transcription activator proteins to one or more TSS of the endogenous lineage-specific factor gene(s) by the one or more gRNA sequences which are complementary to said TSSs, e.g., through binding of at least one of the transcription activator proteins to an aptamer sequence in the gRNA, or through targeting of the catalytically inactive programmable nuclease to the TSS, *e.g.*, because at least one of the transcription activator proteins are fused to the catalytically inactive programmable nuclease. In another embodiment, the expression of the one or more endogenous lineage-specific factor gene is increased by the targeting of transcription activator proteins to the one or more TSSs by a catalytically inactive zinc finger nuclease (ZFN) or transcription activator-like effector nuclease (TALEN) protein.

In one embodiment, the iPSC may be forward programmed into a neuron. Neurons may be defined by their structure and/or function (*e.g.*, neurotransmitter, polarity and signal direction). In a further embodiment, the neuron is a glutamatergic neuron. Such neurons may be characterised by the expression of the glutamate transporter genes VGLUT1 and VGLUT2.

According to this embodiment the one or more gRNA sequences are complementary to the TSSs of endogenous lineage-specific factor genes associated with neurons and the neuronal lineage. Examples of such genes include the endogenous *NEUROG2* (neurogenin 2 or NGN2) gene and the endogenous *NEUROD1* (neuronal differentiation 1) gene. Thus, in one embodiment the one or more gRNA sequences are complementary to the TSS of the endogenous *NEUROG2* gene or the endogenous *NEUROD1* gene. In a further embodiment, the one or more gRNA sequences are complementary to the TSSs of the endogenous *NEUROG2* gene and the endogenous *NEUROD1* gene. In some embodiments, two or more gRNA sequences are inserted into the first or third GSH sites, and each of the gRNA sequences are complementary to either the TSS of the endogenous *NEUROG2* gene or the TSS of the endogenous *NEUROD1* gene.

Thus, in a further aspect of the invention there is provided a method of forward programming an iPSC into a neuron, said method comprising:
(i) targeted insertion of a gene encoding a transcriptional regulator protein into a first genomic safe harbour (GSH) site, wherein the first GSH site is the ROSA26 locus;
(ii) targeted insertion of an inducible cassette into a second GSH site, wherein said inducible cassette comprises a genetic sequence encoding a catalytically inactive programmable nuclease protein and one or more transcription activator proteins operably linked to an inducible promoter, wherein said inducible promoter is regulated by the transcriptional regulator protein, and wherein the second GSH site is the AAVS1 locus; and
(iii) targeted insertion of one or more guide RNA (gRNA) sequences into a third GSH site, wherein said one or more gRNA sequences are complementary to one or more transcription start sites (TSSs) of the endogenous *NEUROG2* gene and/or the TSS of the endogenous *NEUROD1* gene and is operably linked to a constitutive promoter, and wherein the third GSH site is the CLYBL gene.

In a yet further aspect of the invention, there is provided a method of forward programming an iPSC into a neuron, said method comprising:
(i) targeted insertion of a gene encoding a transcriptional regulator protein into a first genomic safe harbour (GSH) site, wherein the first GSH site is the ROSA26 locus;
(ii) targeted insertion of an inducible cassette into a second GSH site, wherein said inducible cassette comprises a genetic sequence encoding a catalytically inactive programmable nuclease protein fused to one or more transcription activator proteins operably linked to an inducible promoter, wherein said inducible promoter is regulated by the transcriptional regulator protein, and wherein the second GSH site is the AAVS1 locus; and
(iii) targeted insertion of one or more guide RNA (gRNA) sequences into a third GSH site, wherein said one or more gRNA sequences are complementary to one or more transcription start sites (TSSs) of the endogenous *NEUROG2* gene and/or the TSS of the endogenous *NEUROD1* gene and is operably linked to a constitutive promoter, and wherein the third GSH site is the CLYBL gene.

In one embodiment of these aspects, the transcriptional regulator protein encoded by the gene inserted into the first GSH site is a tetracycline-responsive transcriptional activator protein (rtTa) and the inducible promoter is a Tet Responsive Element (TRE). According to this embodiment, forward programming of the iPSC into a neuron is performed by supplying exogenous tetracycline or one of its derivatives (*e.g.*, by culturing the iPSC in the presence of tetracycline or a derivative thereof), thereby activating rtTa such that it binds to the TRE of the inducible cassette inserted into the second GSH site and leads to the transcription of the genetic sequence encoding dCas9-VPR.

In further embodiments of these aspects, transcription of the gene encoding the transcriptional regulator protein (*e.g*., rtTA) inserted into the first GSH site is under the control of a CAG promoter, and the constitutive promoter operably linked to the one or more gRNA sequences inserted into the third GSH site is a U6 promoter, such as the human U6 promoter (hU6), such that transcription of the one or more gRNA sequences is under the control of said U6 promoter.

In a yet further embodiment of these aspects, the catalytically inactive programmable nuclease protein is Cas9 and is fused to the transcription activator proteins VP64, p65 and Rta *(i.e.,* the catalytically inactive programmable nuclease is dCas9-VPR).

In a still further embodiment of these aspects, two or more gRNA sequences are inserted into the third GSH site, and are complementary to one or more TSS of the endogenous *NEUROG2* gene and one or more TSS of the endogenous *NEUROD1* gene. In particular, the two or more gRNA sequences complementary to one or more TSS of the endogenous *NEUROG2* gene and one or more TSS of the endogenous *NEUROD1* gene may be comprised in a multiple gRNA array as described herein and the inducible cassette further comprises a sequence encoding a nuclease (*e.g.*, an endonuclease, such as Csy4) which recognises and cleaves cleavable sequences comprised in the multiple gRNA sequence array.

Thus, in another aspect of the invention there is provided a method of forward programming an iPSC into a neuron, said method comprising:
(i) targeted insertion of a gene encoding a transcriptional regulator protein into a first genomic safe harbour (GSH) site, wherein the first GSH site is the ROSA26 locus;
(ii) targeted insertion of an inducible cassette into a second GSH site, wherein said inducible cassette comprises a genetic sequence encoding an endonuclease, a catalytically inactive programmable nuclease protein and one or more transcription activator proteins operably linked to an inducible promoter, wherein said endonuclease recognises and cleaves cleavage sites in a multiple gRNA array, wherein said inducible promoter is regulated by the transcriptional regulator protein, and wherein the second GSH site is the AAVS1 locus; and
(iii) targeted insertion of a multiple gRNA array into a third GSH site, wherein said multiple gRNA array comprises two or more guide RNA (gRNA) sequences separated by two or more cleavable sequences, wherein said one or more gRNA sequences are complementary to one or more transcription start sites (TSSs) of the endogenous *NEUROG2* gene and/or the TSS of the endogenous *NEUROD1* gene and is operably linked to a constitutive promoter, and wherein the third GSH site is the CLYBL gene.

In a still further aspect of the invention, there is provided a method of forward programming an iPSC into a neuron, said method comprising:
(i) targeted insertion of a gene encoding a transcriptional regulator protein into a first genomic safe harbour (GSH) site, wherein the first GSH site is the ROSA26 locus;
(ii) targeted insertion of an inducible cassette into a second GSH site, wherein said inducible cassette comprises a genetic sequence encoding an endonuclease fused to a catalytically inactive programmable nuclease protein fused to one or more transcription activator proteins operably linked to an inducible promoter, wherein said endonuclease recognises and cleaves cleavage sites in a multiple gRNA array, wherein said inducible promoter is regulated by the transcriptional regulator protein, and wherein the second GSH site is the AAVS1 locus; and
(iii) targeted insertion of a multiple gRNA array into a third GSH site, wherein said multiple gRNA array comprises two or more guide RNA (gRNA) sequences separated by two or more cleavable sequences, wherein said one or more gRNA sequences are complementary to one or more transcription start sites (TSSs) of the endogenous *NEUROG2* gene and/or the TSS of the endogenous *NEUROD1* gene and is operably linked to a constitutive promoter, and wherein the third GSH site is the CLYBL gene.

### Cells & Cell Compositions

The methods described herein forward program an iPSC into a somatic cell. iPSCs (induced pluripotent stem cells) are cells that have been reprogrammed to an embryonic stem cell-like state by being forced to express genes and factors important for maintaining the defining properties of embryonic stem cells. In 2006, it was shown that overexpression of four specific transcription factors could convert adult cells into pluripotent stem cells. Oct-3/4 and certain members of the Sox gene family have been identified as potentially crucial transcriptional regulators involved in the induction process. Additional genes including certain members of the Klf family, the Myc family, Nanog, and Lin28, may increase the induction efficiency. Examples of the genes which may be used as reprogramming factors to generate iPSCs include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall4, Esrrb, Tbx3 and Glis1, GATA3, GATA6 and these reprogramming factors may be used singly, or in combination of two or more kinds thereof. In particular, the reprogramming factors may comprise at least the Yamanaka factors, *i.e.,* Oct3/4, Sox2, Klf4 and c-Myc. References herein to "pluripotent" refer to cells which have the potential to differentiate into all types/lineages of cell found in an organism. Multipotent stem cells are able to differentiate into a smaller number of cell types than pluripotent cells, such as only those of closely related cell lineages. Oligopotent stem cells can differentiate into only a few cell types, such as lymphoid or myeloid stem cells. Unipotent cells can produce only one cell type and are thus-lineage-specific but have the ability to self-renew which distinguishes them from non-stem cells (*e.g.*, progenitor cells, which cannot self-renew). iPSCs are of particular interest to the present invention. Thus, it is preferred that cells used in the methods of the invention do not require destruction of an embryo, such as a human embryo. In some embodiments, the cells are not derived from human or animal embryos, *i.e.,* the invention does not extend to any methods which involve the destruction of human or animal embryos.

In one embodiment, the iPSC is from a mammal. In a further embodiment, the mammal is a human. Thus, in a particular embodiment the iPSC is from a human and is thus a human stem cell. In an alternative embodiment, the mammal is a mouse, optionally such that the iPSC is a mouse stem cell.

Methods of preparing induced pluripotent stem cells from mouse are known in the art. Induction of iPSCs typically requires the expression of or exposure to at least one member from Sox family and at least one member from Oct family. Sox and Oct are thought to be central to the transcriptional regulatory hierarchy that specifies ES cell identity. For example, Sox may be Sox-1, Sox-2, Sox-3, Sox- 15, or Sox-18; Oct may be Oct-4. Additional factors may increase the reprogramming efficiency, like Nanog, Lin28, Klf4, or c-Myc; specific sets of reprogramming factors may be a set comprising Sox-2, Oct-4, Nanog and, optionally, Lin-28; or comprising Sox-2, Oct4, Klf and, optionally, c-Myc. In one method, the iPSC may be generated by transfecting cells with transcription factors Oct4, Sox2, c-Myc and Klf4 using viral transduction.

In one embodiment, the iPSC is derived from somatic or germ cells of a patient, such as a patient to be treated or subject in need of treatment. Such use of autologous cells would remove the need for matching cells to a recipient. Alternatively, commercially available iPSCs may be used, such as those available from WICELL (WiCell Research Institute, Inc, Wisconsin, US). Alternatively, the cells may be a tissue-specific stem cell which may also be autologous or donated.

In some aspects of the invention, there is provided a cell obtained by the methods described herein. In a particular aspect, there is provided a forward programmed somatic cell obtained by the methods described herein. Thus, in certain aspects of the invention there is provided a cell (*e.g*., a forward programmed somatic cell) obtained by the methods of forward programming an iPSC comprising targeted insertion into at least two (*e.g*., two or three) different GSH sites as described herein. In a further aspect, there is provided a cell (*e.g.*, a forward programmed somatic cell) obtained by the methods of forward programming an iPSC comprising targeted insertion into three different GSH sites as described herein.

In a yet further aspect of the invention, there is provided a cell with a modified genome that comprises:
(i) an inserted genetic sequence encoding a transcriptional regulator protein at a first genomic safe harbour (GSH) site;
(ii) an inserted inducible cassette comprising a genetic sequence encoding a catalytically inactive programmable nuclease protein and one or more transcription activator proteins operably linked to an inducible promoter at a second GSH site, wherein said inducible promoter is regulated by the transcriptional regulator protein; and
(iii) an inserted genetic sequence encoding one or more gRNA sequences operably linked to a constitutive promoter at a third GSH site, wherein said one or more gRNA sequences are complementary to one or more TSS of an endogenous lineage-specific factor gene,
wherein the first, second and third GSH sites are different.

In another aspect, there is provided a forward programmed somatic cell with a modified genome that comprises:
(i) an inserted genetic sequence encoding a transcriptional regulator protein at a first genomic safe harbour (GSH) site;
(ii) an inserted inducible cassette comprising a genetic sequence encoding a catalytically inactive programmable nuclease protein and one or more transcription activator proteins operably linked to an inducible promoter at a second GSH site, wherein said inducible promoter is regulated by the transcriptional regulator protein; and
(iii) an inserted genetic sequence encoding one or more gRNA sequences operably linked to a constitutive promoter at a third GSH site, wherein said one or more gRNA sequences are complementary to one or more TSS of an endogenous lineage-specific factor gene,
wherein the first, second and third GSH sites are different.

In an alternative aspect, there is provided a cell (*e.g.*, a forward programmed somatic cell) with a modified genome that comprises:
(i) an inserted genetic sequence encoding a transcriptional regulator protein and one or more guide RNA (gRNA) sequences at a first genomic safe harbour (GSH) site, wherein said one or more gRNA sequences are complementary to one or more transcription start sites (TSSs) of an endogenous lineage-specific factor gene and is operably linked to a constitutive promoter; and
(ii) an inserted inducible cassette comprising a genetic sequence encoding a catalytically inactive programable nuclease protein and one or more transcription activator proteins operably linked to an inducible promoter at a second GSH site, wherein said inducible promoter is regulated by the transcriptional regulator protein;
wherein the first and second GSH sites are different.

In a particular aspect of the invention, there is provided a neuron forward programmed from an iPSC with a modified genome that comprises:
(i) an inserted genetic sequence encoding a transcriptional regulator protein at a first genomic safe harbour (GSH) site, wherein the first GSH site is the ROSA26 locus;
(ii) an inserted inducible cassette comprising a genetic sequence encoding a catalytically inactive programmable nuclease protein and one or more transcription activator proteins operably linked to an inducible promoter at a second GSH site, wherein said inducible promoter is regulated by the transcriptional regulator protein, and wherein the second GSH site is the AAVS1 locus; and
(iii) an inserted genetic sequence encoding one or more guide RNA (gRNA) sequences operably linked to a constitutive promoter at a third GSH site, wherein said one or more gRNA sequences are complementary to one or more transcription start sites (TSSs) of the endogenous *NEUROG2* gene and/or the TSS of the endogenous *NEUROD1* gene, and wherein the third GSH site is the CLYBL gene.

In one embodiment of this particular aspect, the transcriptional regulator protein encoded by the genetic sequence inserted at the first GSH site may be tetracycline-responsive transcriptional activator protein (rtTa) and the inducible promoter inserted as part of the inducible cassette inserted at the second GSH site may be a Tet Responsive Element (TRE).

In further embodiments of this particular aspect, the genetic sequence encoding a transcriptional regulator protein inserted at the first GSH site comprises a CAG promoter, and the constitutive promoter operably linked to the one or more gRNA sequences inserted at the third GSH site is a U6 promoter, such as the human U6 promoter (hU6).

In a yet further embodiment of this particular aspect, the catalytically inactive programmable nuclease protein is dCas9 and is fused to the transcription activator proteins VP64, p65 and Rta *(i.e.,* the catalytically inactive programmable nuclease is dCas9-VPR).

In a still further embodiment of this particular aspect, the genetic sequence inserted at the third GSH site encodes two or more gRNA sequences which are complementary to one or more TSS of the endogenous *NEUROG2* gene and one or more TSS of the endogenous *NEUROD1* gene. According to this embodiment, the two or more gRNA sequences complementary to one or more TSS of the endogenous *NEUROG2* gene and one or more TSS of the endogenous *NEUROD1* gene may be comprised in a multiple gRNA array as described herein and the inducible cassette may further comprise a sequence encoding a nuclease (*e.g*., an endonuclease, such as Csy4) which recognises and cleaves cleavable sequences comprised in the multiple gRNA sequence array.

In one embodiment, the cell (*e.g*., the forward programmed somatic cell) obtained by the methods described herein is provided in a pharmaceutical composition. In another embodiment, the cell (*e.g*., the forward programmed somatic cell) as described herein is provided in a pharmaceutical composition.

In one embodiment, cells described here may be used for the manufacturing of biologics, such as antibodies or for viruses used in cell and gene therapy. In this instance, the cell may be an iPSC, a CHO cell or a HEK293T cell.

Pharmaceutical compositions may include cells as described herein in combination with one or more pharmaceutically or physiologically acceptable carrier, diluents, or excipients. Such compositions may include buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (*e.g.*, aluminium hydroxide); and preservatives. Cryopreservation solutions which may be used in the pharmaceutical compositions of the invention include, for example, DMSO.

For purposes of manufacture, distribution, and use, the cells described herein may be supplied in the form of a cell culture or suspension in an isotonic excipient or culture medium, optionally frozen to facilitate transportation or storage.

It will be readily understood that the methods described hereinbefore, or any/all steps thereof, may be performed *in vivo, ex vivo* or *in vitro.* In a particular embodiment, the methods described hereinbefore, or any/all steps thereof, are performed *in vitro,* such as *ex vivo.*

### Uses, Therapeutic Uses & Methods of Treatment

The cells produced according to any of the methods described herein have applications in basic and medical research, diagnostic and therapeutic methods. The cells may be used *in vitro* to study cellular development, provide test systems for new drugs, enable screening methods to be developed, scrutinise therapeutic regimens, provide diagnostic tests and the like. These uses form part of the present invention. Alternatively, the cells may be transplanted into a human or animal patient for diagnostic or therapeutic purposes. The use of the cells in therapy is also included in the present invention.

Thus, in one aspect of the invention, there is provided the cell obtained by the methods described herein for use in therapy. In a further aspect there is provided a forward programmed somatic cell obtained by the methods described herein for use in therapy. In certain embodiments, the cell (*e.g.,* the forward programmed somatic cell) is the cell (*e.g.,* the forward programmed somatic cell) described hereinbefore.

In another aspect of the invention, there is provided a method of treatment comprising administering to a patient or subject in need thereof the cell obtained by the methods described herein. In a further aspect of the invention, there is provided a method of treatment comprising administering to a patient or subject in need thereof the forward programmed somatic cell obtained by the methods described herein. In one embodiment, the patient or subject in need of treatment is suffering from a disease or disorder. In another embodiment, the patient or subject in need thereof requires a cosmetic treatment.

In a further aspect, there is provided the neuron forward programmed from an iPSC as described herein for use in therapy. In another aspect, there is provided a method of treatment comprising administering to a patient or subject in need thereof the neuron forward programmed from an iPSC as described herein.

In one embodiment, the disease or disorder to be treated is selected from one or more of: cancer, a neurological disorder, an inflammatory disease, an autoimmune disease, and/or a chronic infectious disease. In a further embodiment, the therapy is a therapy/treatment for cancer, a neurological disorder, an inflammatory disease, an autoimmune disease, and/or a chronic infectious disease.

Unless defined otherwise, all technical and scientific terms used herein have the meaning commonly understood by a person skilled in the art to which this invention belongs. As used herein, the term "about" when used herein includes up to and including 10% greater and up to and including 10% lower than the value specified, suitably up to and including 5% greater and up to and including 5% lower than the value specified, especially the value specified. The term "between" as used herein includes the values of the specified boundaries.

It will be understood that all embodiments described herein may be applied to all aspects of the invention and *vice versa,* and such combinations would be readily apparent from the description provided herein and to those skilled in the art.

Other features and advantages of the present invention will be apparent from the description provided herein. It should be understood, however, that the description and the specific examples while indicating preferred embodiments of the invention are given by way of illustration only, since various changes and modifications will become apparent to those skilled in the art.

### CLAUSES

A set of clauses defining the invention and its preferred aspects is as follows:
1. A method of forward programming an induced pluripotent stem cell (iPSC) into a somatic cell, said method comprising:
   (i) targeted insertion of a gene encoding a transcriptional regulator protein into a first genomic safe harbour (GSH) site;
   (ii) targeted insertion of an inducible cassette into a second GSH site, wherein said inducible cassette comprises a genetic sequence encoding a catalytically inactive programable nuclease protein and one or more transcription activator proteins operably linked to an inducible promoter, and wherein said inducible promoter is regulated by the transcriptional regulator protein;
   (iii) targeted insertion of one or more guide RNA (gRNA) sequences into a third GSH site, wherein said one or more gRNA sequences are complementary to one or more transcription start sites (TSSs) of an endogenous lineage-specific factor gene and is operably linked to a constitutive promoter,
   wherein the first, second and third GSH sites are different.
2. The method of clause 1, wherein the one or more gRNA sequences targets the catalytically inactive programmable nuclease protein and one or more transcription activator proteins to the TSS of the endogenous lineage-specific factor gene, and/or
   wherein the one or more transcription activator proteins activates or increases transcription at the TSS of the endogenous lineage-specific factor gene.
3. The method of clause 1 or clause 2, wherein the one or more transcription activator proteins is fused to the catalytically inactive programable nuclease protein.
4. The method of any one of clauses 1 to 3, wherein the one or more transcription activator proteins is selected from one or more of: VP16, VP64, p65, Rta, MS2 and HSF1, such as VP64, and/or
   wherein the one or more transcription activator proteins is a combination of VP64, p65 and Rta or a combination of MS2, p65 and HSF1, such as wherein the transcription activator protein is a VP64-p65-Rta fusion protein or a MS2-p65-HSF1 fusion protein.
5. The method of any one of clauses 1 to 4, wherein two or more gRNA sequences are inserted into the third GSH site, and wherein each of the gRNA sequences are complementary to alternative or different TSSs of the endogenous lineage-specific factor gene, or are complementary to more than one alternative sequences of the TSS of the endogenous lineage-specific factor gene.
6. The method of any one of clauses 1 to 5, wherein the one or more gRNA sequences comprise an MS2 aptamer sequence, optionally wherein the MS2 aptamer sequence recruits an MS2-containing fusion protein to the TSS of the endogenous lineage-specific factor gene, such as an MS2-p65-HSF1 fusion protein.
7. The method of any one of clauses 1 to 6, wherein the catalytically inactive programable nuclease protein is catalytically inactive Cas9, optionally comprising point mutations in the RuvCI and HNH nuclease domains, such as D10A and H840A compared to the wild-type sequence of Cas9.
8. The method of any one of clauses 1 to 7, wherein the activity of the transcriptional regulator protein is controlled by an exogenous substance.
9. The method of any one of clauses 1 to 8, wherein the transcriptional regulator protein is constitutively expressed.
10. The method of any one of clauses 1 to 9, wherein the transcriptional regulator protein is selected from any one of: a tetracycline-responsive transcriptional activator protein (rtTa), a tetracycline repressor (TetR), VgEcR synthetic receptor, a cumate repressor (CymR) or a hybrid transcriptional regulator protein comprising a DNA binding domain from the yeast GAL4 protein, a truncated ligand binding domain from the human progesterone receptor and an activation domain from the human NF-κB,
   optionally wherein the transcriptional regulator protein is rtTA and its activity is controlled by tetracycline of a derivative thereof, such as doxycycline, and optionally wherein the inducible promoter includes a tetracycline-response element (TRE).
11. The method of any one of clauses 1 to 10, wherein the first, second and third GSH sites are selected from any three of: the hROSA26 locus, the AAVS1 locus, the CLYBL gene and/or the CCR5 gene, such as wherein the first GSH site is the hROSA26 locus, the second GSH site is the AAVS1 locus and the third GSH site is the CLYBL gene.
12. The method of any one of clauses 1 to 11, wherein insertion of the gene encoding a transcriptional regulator protein into the first GSH site occurs on both chromosomes of the cell, insertion of the inducible cassette into the second GSH site occurs on both chromosomes of the cell and/or insertion of the one or more gRNA sequence into the third GSH site occurs on both chromosomes of the cell.
13. The method of any one of clauses 1 to 12, wherein the one or more gRNA sequences are complementary to the TSS of the endogenous *NEUROG2* gene and/or the endogenous *NEUROD1* gene, and wherein the iPSC is forward programmed into a neuron.
14. A method of forward programming an iPSC into a neuron, said method comprising:
   (i) targeted insertion of a gene encoding a transcriptional regulator protein into a first genomic safe harbour (GSH) site, wherein the first GSH site is the ROSA26 locus;
   (ii) targeted insertion of an inducible cassette into a second GSH site, wherein said inducible cassette comprises a genetic sequence encoding a catalytically inactive programmable nuclease protein and one or more transcription activator proteins operably linked to an inducible promoter, wherein said inducible promoter is regulated by the transcriptional regulator protein, and wherein the second GSH site is the AAVS1 locus; and
   (iii) targeted insertion of one or more guide RNA (gRNA) sequences into a third GSH site, wherein said one or more gRNA sequences are complementary to one or more transcription start sites (TSSs) of the endogenous *NEUROG2* gene and/or the TSS of the endogenous *NEUROD1* gene and is operably linked to a constitutive promoter, and wherein the third GSH site is the CLYBL gene.
15. A cell obtained by the method of any one of clauses 1 to 14.
16. A cell with a modified genome that comprises:
   (i) an inserted genetic sequence encoding a transcriptional regulator protein at a first genomic safe harbour (GSH) site;
   (ii) an inserted inducible cassette comprising a genetic sequence encoding a catalytically inactive programmable nuclease protein and one or more transcription activator proteins operably linked to an inducible promoter at a second GSH site, wherein said inducible promoter is regulated by the transcriptional regulator protein; and
   (iii) an inserted genetic sequence encoding one or more gRNA sequences operably linked to a constitutive promoter at a third GSH site, wherein said one or more gRNA sequences are complementary to one or more TSS of an endogenous lineage-specific factor gene,
   wherein the first, second and third GSH sites are different.
17. A neuron forward programmed from an iPSC with a modified genome that comprises:
   (i) an inserted genetic sequence encoding a transcriptional regulator protein at a first genomic safe harbour (GSH) site, wherein the first GSH site is the ROSA26 locus;
   (ii) an inserted inducible cassette comprising a genetic sequence encoding a catalytically inactive programmable nuclease protein and one or more transcription activator proteins operably linked to an inducible promoter at a second GSH site, wherein said inducible promoter is regulated by the transcriptional regulator protein, and wherein the second GSH site is the AAVS1 locus; and
   (iii) an inserted genetic sequence encoding one or more guide RNA (gRNA) sequences operably linked to a constitutive promoter at a third GSH site, wherein said one or more gRNA sequences are complementary to one or more transcription start sites (TSSs) of the endogenous *NEUROG2* gene and/or the TSS of the endogenous *NEUROD1* gene, and wherein the third GSH site is the CLYBL gene.
18. The cell of any one of clauses 15 to 17 for use in therapy.
19. The cell of any one of clauses 15 to 17 for use in the treatment of cancer, a neurological disorder, an inflammatory disease, an autoimmune disease and/or a chronic infectious disease.

The invention will now be described using the following, non-limiting examples:

### EXAMPLES

### Example 1 - Forward Programming of Glutamatergic Neurons Using Insertion of a Single gRNA

### Materials and Methods

### Cell Line Engineering (Single gRNA)

An iPSC line was cultured at all times in StemFlex media (Gibco, A3349401) on Vitronectin (Stem Cell Technologies, 07180) coated plates. Editing of the safe harbour loci was performed subsequentially. All three loci were edited using CRISPR/Cas9 technology. A sgRNA which introduces a DNA double-strand break in close proximity to the chosen insertion site was selected. Cells were nucleofected with two plasmids using a Lonza 4D-Nucleofector - a plasmid expressing Cas9 and the corresponding sgRNA and a plasmid containing the insert sequence flanked by homology arms. The PAM sequence was mutated to prevent the re-cutting of Cas9.

### Edit 1:

ROSA26 locus with sgRNA: GTCGAGTCGCTTCTCGATTA (SEQ ID NO: 1)
Donor template: 5' Homology arm-Splice acceptor site-NeoR-CAG-rtTA-3' Homology arm
Selection: Neomycin

### Edit 2:

AAVS1 locus with sgRNA: GTCCCTAGTGGCCCCACTGTG (SEQ ID NO: 2)
Donor template: 5' Homology arm-Splice acceptor site-PuroR-TRE3G-dCas9-VPR-3' Homology arm
Selection: Puromycin
Puromycin Resistance gene was knocked-out before editing of the CLYBL locus (**Edit 3** - Puromycin sgRNA: CACGCGCCACACCGTCGATC (SEQ ID NO: 3))

### Edit 4:

CLYBL locus with sgRNA: GTTCGTCTAGAAAGAAAGACT (SEQ ID NO: 4)
Donor template: 5' Homology arm-Splice acceptor site-PuroR-insulator-hU6-sgRNA NEUROG2-insulator-3' Homology arm (NEUROG2 sgRNA: GGCCAAGGGAGGGGGAA AGG (SEQ ID NO: 5))
Selection: Puromycin

For each nucleofection cells were dissociated as single cells with Accutase (Merck, A6964) prior to a 3h treatment with CloneR (Stem Cell Technologies, 05888) and recovered after the exposure to the electric pulse in StemFlex media supplemented with CloneR for 48h. After five days of recovery, cells harbouring the correct integration were selected with the respective antibiotic. Once selection has reached a sufficient efficiency, cells were dissociated as single cells with Accutase. To obtain monoclonal lines, cells were seeded at a low density to allow outgrowth of colonies originating from a single cell.

Engineered lines were submitted to two rounds of genotyping by PCR. The first round of genotyping was performed on crude lysate. Once clones with the correct integration were identified, cells were expanded, and genomic DNA was isolated for a second round of genotyping. PCRs for correct integration of 5' and 3' homology arms as well as the insert sequence. In addition, PCRs were performed to confirm if clones are heterozygous or homozygous for the intended integration. Before the CLYBL locus was edited the Puromycin resistance gene in the AAVS1 locus was knocked out. The Puromycin knock-out was confirmed by the loss of cell viability upon Puromycin treatment.

### Genotyping

Genotyping PCRs are performed using 10ng of genomic DNA input. Primers are designed to span different regions of the hU6-sgRNA cassette as shown in **Figure 2****.** For all PCRs the OneTaq Polymerase Master Mix was used (NEB; M0482L).

### Primer Sequences

Heterozygous/Homozygous (expected size: 2069 bp; Figure 2A):
**PR2284 Fwd primer:** CTGTCCAGTCCCCATTTGAG (SEQ ID NO: 6)
**PR2264 Rev primer:** CAGATGGAGCAGTGGATGAC (SEQ ID NO: 7)

CLYBL 5' integration (expected size: 1116 bp; Figure 2B):
**PR2284 Fwd primer:** CTGTCCAGTCCCCATTTGAG (SEQ ID NO: 8)
**PR2285 Rev primer:** CACGTCACCGCATGTTAGAA (SEQ ID NO: 9)

CLYBL 3' integration (expected size: 949 bp; Figure 2C):
**PR2282 Fwd primer:** CGCGGATATGAGTCTGTCTAAG (SEQ ID NO: 10)
**PR2149 Rev primer:** AGACACTTTACGGCTCTGTTGG (SEQ ID NO: 11)

On-target integration (expected size: 1146 bp; Figure 2D):
**PR2079 Fwd primer:** TTTCCCATGATTCCTTCATATTTGC (SEQ ID NO: 12)
**PR2232 Rev primer:** CAAATCAAACATGGCTCAGTTGTG (SEQ ID NO: 13)

Backbone integration (expected size: 519 bp):
**PR2018 Fwd primer:** CCGCCTCCATCCAGTCTATT (SEQ ID NO: 14)
**PR2017 Rev primer:** GTGCACGAGTGGGTTACATC (SEQ ID NO: 15)

### Immunofluorescence Staining

Prior to immunocytochemistry, cells were washed in PBS (Gibco, 12549079) to remove debris and medium and fixed in 4% paraformaldehyde (Science Services, 15710; diluted in PBS) for 15 minutes at room temperature followed by two 5-minute washes in PBS and subsequently kept in PBS at +4°C for temporary storage until immunofluorescence staining is performed.

Cells were permeabilised and blocked in 0.1% Triton X-100 (Sigma, 9036-19-5) in PBS containing 4% of goat serum (Sigma, G9023-10mL) for 45 minutes at room temperature followed by two washes in PBS. Next, cells were incubated with primary antibodies (MAPT; Abcam, ab76128) diluted 1:250 in blocking solution (PBS with 0.1% Triton X-100 and 4% goat serum) at 4°C overnight. After incubation, cells were washed with PBS supplemented with 0.1% Triton X-100 three times for five minutes. Fluorescently labelled secondary antibodies (Goat anti-Rabbit IgG (H+L) Highly Cross-Adsorbed Secondary Antibody, Alexa Fluor Plus 555, ThermoFisher, A-32732) diluted 1:500 in blocking solution were added and allowed to incubate at room temperature for 1 hour. In a last step, cells were washed for five minutes with PBS, followed by a 10-minute incubation in 1µg/ml of DAPI (4',6-diamidino-2-phenylindole) and additional washes with PBS. Cells are kept in PBS at 4°C until imaging is completed. Cells were imaged with the Evos FL microscope.

### Results

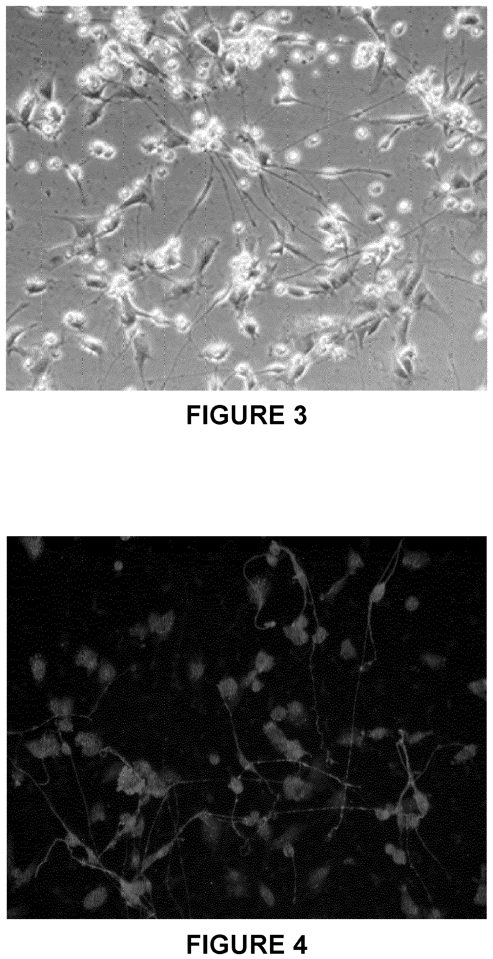

To forward program glutamatergic neurons from iPSCs with a modified genome comprising three GSH site insertions as described herein, cells were seeded as single cells on Geltrex in StemFlex media supplemented with ROCK inhibitor and doxycycline. Doxycycline was present in all media until day 7 of differentiation. After 24 hours, the media was changed to a DMEM/F12-based induction media containing Glutamax, MEM NEAA, N2 supplement and 2-Mercaptoethanol. The specification of cells to become glutamatergic neurons was induced by neurobasal media containing B27, NT-3, BDNF, Glutamax and 2-Mercaptoethanol on a PDL/Geltrex coating. After seven days of differentiation, cells with neuronal morphology were observed **(****Figure 3****).** Importantly, these cells expressed MAP2 **(****Figure 4****),** which represents a key neuronal marker gene and therefore shows successful forward programming into neurons.

### Example 2 - Forward Programming of Glutamatergic Neurons Using a Multiple gRNA Array

### Materials and Methods

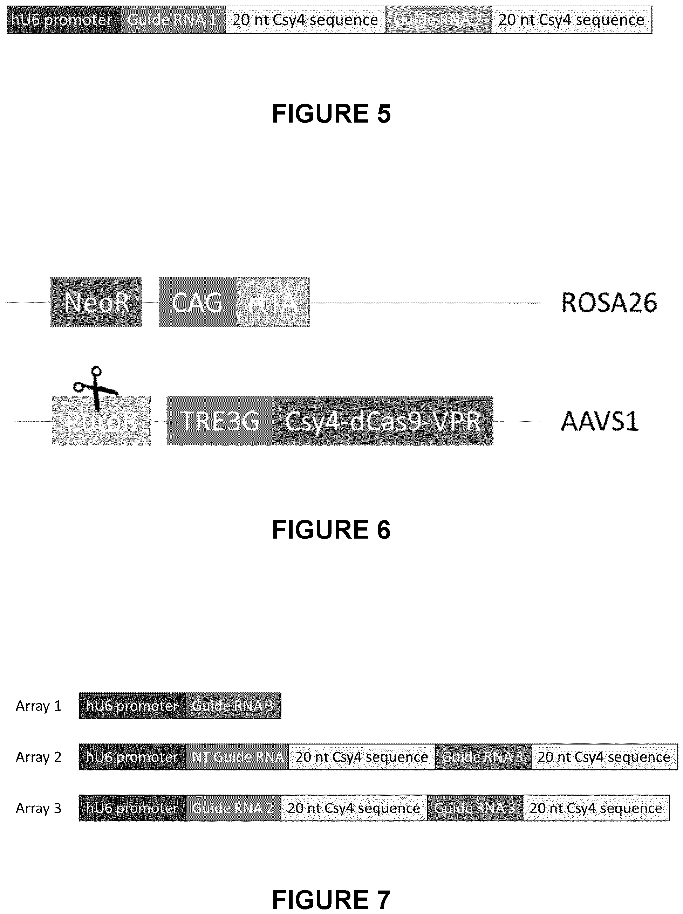

To test if higher forward programming efficiency using CRISPRa could be achieved, a cell line harbouring Csy4-dCas9-VPR in AAVS1 was generated. Csy4 is a bacterial DNA endonuclease which herein is linked N-terminally via T2A to dCas9-VPR. Csy4 recognises a specific 20nt sequence which is cleaved after the 20^{th} nucleotide. This allows the delivery of multiple guide RNAs using recombinant lentiviruses. The guides are synthesised as a Csy4-cleavable array **(****Figure 5****).**

### Lentiviral Preparation

LentiX cells (Takara Bio, 632180) were transfected with second generation lentiviral plasmids. After 24h of incubation at 37°C and 5% CO₂ media was changed. On the next day, the supernatant containing lentiviral particles was harvested and stored at 4°C. Media was replenished and cells cultured for another 24h until the second media harvest. Supernatants from both days are combined and concentrated by PEG precipitation (System Biosciences, LV825A-1).

### Lentiviral Transduction of hiPSCs

For each transduction cells were dissociated as single cells with Accutase (Merck, A6964) and transduced with the lentivirus using Polybrene (Santa Cruz, A1110501) at a MOI of 0.1. After recovery in StemFlex media (ThermoFisher, A3349401) supplemented with RevitaCell (ThermoFisher, A2644501) for 24h, selection with Puromycin (Invivogen, ant-pr-1) was started. After five days of selection, cells were dissociated as single cells with Accutase and forward programming was started.

### Cell Line Engineering (Multiple gRNA Array Insertion)

The configuration shown in **Figure 6** allows the Doxycycline-mediated upregulation of Csy4-dCas9-VPR. Upon expression, Csy4 will process the guide array and dCas9-VPR will recruit the cleaved gRNA sequences which are constitutively expressed from the U6 promoter. This will lead to the upregulation of the target gene(s), thus driving forward programming/ differentiation of iPS cells into the desired target cell type. Three different guides were tested in combination **(****Figure 7****):**
NT (non-targeting) Guide RNA: GCGCGACGCTAATCCGTCGA (SEQ ID NO: 16)
Guide RNA 2: GTGGTATATAAGGGGTTTTA (SEQ ID NO: 17)
Guide RNA 3: GGCCAAGGGAGGGGGAAAGG (SEQ ID NO: 18)

Array 1: 
ggccaagggagggggaaagggtttcagagctatgctggaaacagcatagcaagttgaaataaggctagtccgttatcaacttg
aaaaagtggcaccgagtcggtgcttttttt (SEQ ID NO: 19)
Array 2: 
gcgcgacgctaatccgtcgagtttaagagctaagctggaaacagcatagcaagtttaaataaggctagtccgttatcaacttggc
cGCTTTAAGGCCGGTCCTAGCAAggccaagtggcaccgagtcggtgcGTTCACTGCCGTATAGGC
AGggccaagggagggggaaagggtttcagagctatgctggaaacagcatagcaagttgaaataaggctagtccgttatcaa
cttgaaaaagtggcaccgagtcggtgcttttttt (SEQ ID NO: 20)
Array 3:

gtggtatataaggggttttagtttaagagctaagctggaaacagcatagcaagtttaaataaggctagtccgttatcaacttggccG
CTTTAAGGCCGGTCCTAGCAAggccaagtggcaccgagtcggtgcGTTCACTGCCGTATAGGCAG

ggccaagggagggggaaagggtttcagagctatgctggaaacagcatagcaagttgaaataaggctagtccgttatcaacttg
aaaaagtggcaccgagtcggtgcttttttt (SEQ ID NO: 21)

Lower case nucleotides = CRISPRa gRNA sequences (non-targeting/*NEUROG2*-targeting); underlined lower case nucleotides = tcRNA sequence; uppercase nucleotides = capture sequence 1; uppercase bold nucleotides = Csy4 20nt recognition site.

All three guide arrays were cloned into a lentiviral vector for lentivirus production. Cells were transduced with an MOI of ~0.1 to guarantee single integration of the lentiviral cassette. Cells were selected with Puromycin to eliminate non-transduced cells (~80% selection efficiency).

### Results

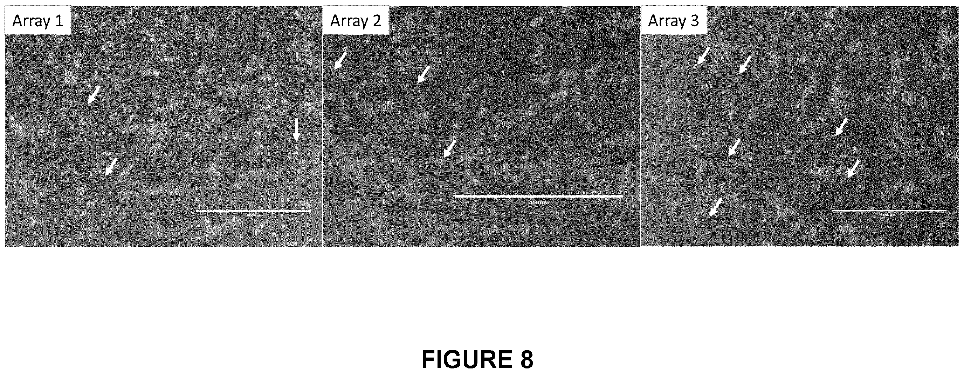

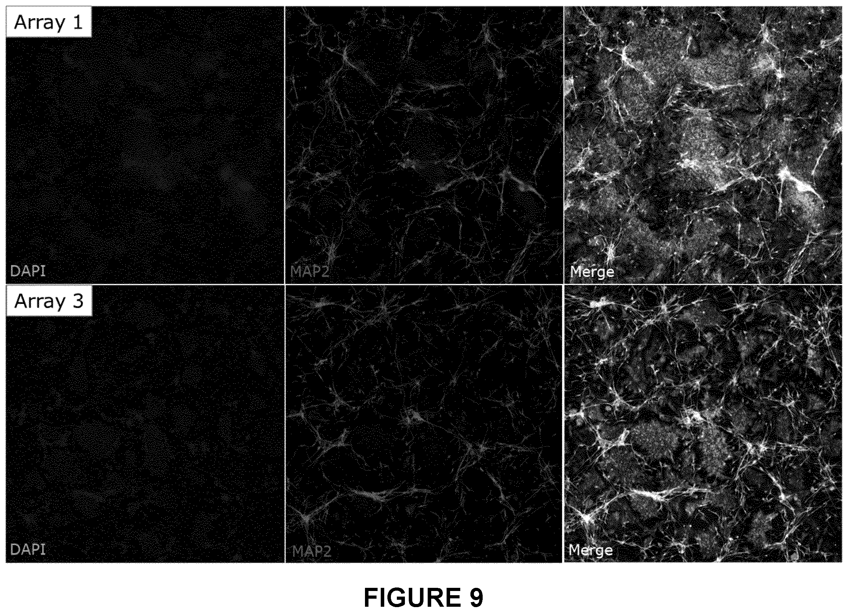

To forward program glutamatergic neurons from iPSCs with a modified genome comprising inserted multiple gRNAs as described herein, single cells were seeded on Geltrex in StemFlex media supplemented with ROCK inhibitor and doxycycline. Doxycycline is present in all media until day 7 of differentiation. After 24h, the media was changed to a DMEM/F12-based induction media containing Glutamax, MEM NEAA, N2 supplement and 2-Mercaptoethanol. The specification of cells to become glutamatergic neurons is induced by neurobasal media containing B27, NT-3, BDNF, Glutamax and 2-Mercaptoethanol on a PDL/Geltrex coating. After seven days of differentiation, cells with neuronal morphology were observed (**Figure 8**). Array 3 showed highest differentiation efficiency based on morphology. Importantly, these cells expressed MAP2 (**Figure 9**), which represents a key neuronal marker gene. While array 1 and array 2 both show MAP2 positive neurons, cells transduced with array 1 have more non-differentiated cells which are still proliferating and outgrowing the neuronal culture.

### Further Sequences:

CLYBL-hU6-sgRNA NEUROG2 (SEQ ID NO: 22):

GAACTCTGCCACCTAATTTAGGATCCCAGACTCTCCTACTGGAGACACAGGTCCTTAG
TGACAGTCTGCACCACCATTCAGACAAGTCAGTAGGGCCATCTTAGATCATCCAGCC
CTAGTCAAGCCACCAGATAACTGTACCCACATAAGTGACCCCTGGCGAGACGAGCAG
GAGAATCATGCCAATGGGCCAATATACATTCTGACCCACAGTTTCATAATAAAATAAA
ATGGTTGTGGTTGTAAGCCACTATGTTTCAGAGTGGTTTGTTACACAGCAATAAATAA
CTAATATAGTAGGCATACCATCAAGTCCAAAGTAGGTAGAGAAGAATGTAAATAGCA
GAGCAAAACAGCATGACTGGTGGCTGGGAGGCTTAAAACTGGGACAGGATCAGAGT
CATGAAAGAAGTCAAAGAAATGGTTCAGAAGTAAGGCTGAGACTGACTTACAAAAGC
TGAAAGTCCCTTTAAGTTGGTGTTTGGTGCATTGGCAGGGGCAGGTATGGTGACTTAA
AAGAGCCATGCTCAACAAGATCAAGCACAACACAATCACGGGTCACCCCAGCAGAC

CTTAGCGAGTCTAGCCATTTCTTTGGTGGTGGTCACAGTCATGCTTCAGCCCAGTTTC
CACTTGGACAAATGGTACATATTTTCAATGAGATGAAAATTAAGATACAATCCATGTG
CTCAGAGAGTGATCACAGCTCTGACTAAACACTGTGCCCCAAAGTGTTGAGGAATTG
GGAAAACCTAGCTGAGTTAGTGATCTCTTTTCTGTTACAATAAAGCTCATAATGAAAA
TTAGCCTTCTTTGTTCTTCCGGTACGGCAAGCTTCTGACCTCTTCTCTTCCTCCCACAG
GGCCTCGAGAGATCTGGCAGCGGAgagggcagaggaagtcttctaacatgcggtgacgtggaggaga
atcccggccctAGGCTCGAGATGACCGAGTACAAGCCCACGGTGCGCCTCGCCACCCGC
GACGACGTCCCCAGGGCCGTACGCACCCTCGCCGCCGCGTTCGCCGACTACCCCGC
CACGCGCCACACCGTCGATCCGGACCGCCACATCGAGCGGGTCACCGAGCTGCAAG
AACTCTTCCTCACGCGCGTCGGGCTCGACATCGGCAAGGTGTGGGTCGCGGACGAC
GGCGCCGCGGTGGCGGTCTGGACCACGCCGGAGAGCGTCGAAGCGGGGGCGGTGT
TCGCCGAGATCGGCCCGCGCATGGCCGAGTTGAGCGGTTCCCGGCTGGCCGCGCAG
CAACAGATGGAAGGCCTCCTGGCGCCGCACCGGCCCAAGGAGCCCGCGTGGTTCCT
GGCCACCGTCGGCGTCTCGCCCGACCACCAGGGCAAGGGTCTGGGCAGCGCCGTCG
TGCTCCCCGGAGTGGAGGCGGCCGAGCGCGCCGGGGTGCCCGCCTTCCTGGAGACC
TCCGCGCCCCGCAACCTCCCCTTCTACGAGCGGCTCGGCTTCACCGTCACCGCCGAC
GTCGAGGTGCCCGAAGGACCGCGCACCTGGTGCATGACCCGCAAGCCCGGTGCCTG
ATCTAGAGGGCCCGTTTAAACCCGCTGATCAGCCTCGActgtgccttctagttgccagccatctgtt
gtttgcccctcccccgtgccttccttgaccctggaaggtgccactcccactgtcctttcctaataaaatgaggaaattgca
tcgcattgtctgagtaggtgtcattctattctggggggtggggtggggcaggacagcaagggggaggattgggaaga
caatagcaggcatgctggggatgcggtgggctctatggTCACTCGAGTtagacagactcatatccgcgggagct
cacggggacagccccccccccaaagcccccagggctgtaattgcgttcctctcccgctagggggcggcagcgagc
cgcccagggctccgctccggcccgacgctccccccgcatccccgagccggagcccgcagcgtgcggggacagc
ccggcacggggaaggtggcacgggatcgctttcctctgaacgcttctcgctgctctttgagcctgcagacacctgggg
ggatacggggaaaaagctttatctagatccgcgggagctcacggggacagcccccccccaaagcccccagggctg
taattgcgttcctctcccgctagggggcagcagcgagccgcccggggctccgctccggcccgacgctccccccgc
atccccgagccggagcccgcagcgtgcggggacagcccggcacggggaaggtggcacgggatcgctttcctctg
aacgcttctcactgctctttgagcctgcagacacctggggggatacggggaaaaagctttatcgctctgtcgcaTTTC
CCATGATTCCTTCATATTTGCATATACGATACAAGGCTGTTAGAGAGATAATTAGAATTA
ATTTGACTGTAAACACAAAGATATTAGTACAAAATACGTGACGTAGAAAGTAATAATTTC
TTGGGTAGTTTGCAGTTTTAAAATTATGTTTTAAAATGGACTATCATATGCTTACCGTAAC
TTGAAAGTATTTCGATTTCTTGGCTTTATATATCTTGTGGAAAGGACGAAACACCggccaag
ggagggggaaagggtttcagagctatgctggaaacagcatagcaagttgaaataaggctagtccgttatcaacttgaaaaagt
ggcaccgagtcggtgctttttttCTTGACAGTACTcctactggcggacagagcgataaagctttttccccgtatccccc
caggtgtctgcaggctcaaagagcagtgagaagcgttcagaggaaagcgatcccgtgccaccttccccgtgccggg
ctgtccccgcacgctgcgggctccggctcggggatgcggggggagcgtcgggccggagcggagccccgggcgg
ctcgctgctgccccctagcgggagaggaacgcaattacagccctgggggctttgggggggggctgtccccgtgagc

tcccgcggatctagataaagctttttccccgtatccccccaggtgtctgcaggctcaaagagcagcgagaagcgttca
gaggaaagcgatcccgtgccaccttccccgtgccgggctgtccccgcacgctgcgggctccggctcggggatgcg
gggggagcgtcgggccggagcggagccccgggcggctcgctgctgccccctagcgggagaggaacgcaattac
agccctgggggctttgggggggggctgtccccgtgagctcccgcggatatgagtctgtctaagtgCTTAAGTCG
ACTTACTCGACTTTCAGACTGTTATTTCTTGACTTTTAAATGTAAGATATTATCTACTGA
CTTCCTTCTATGTAAGATGAGGATTGAGCTCTCTTACCCTTCTCCCATTTCCTCATCCT
TCCAACATAAATATATTTTGGGATTATATCAACATTCAATGTTACTTAAAGTGACCTTG
TAAATATTTTCACAACTGAGCCATGTTTGATTTGTATACTTATGTTTACTTTACTGTTTTT
CCTGAAGTTAATAATTGCCTTGAATTTATTTATTTCTTTAAAAATGTTTCATTACTCAGG
ACTGTAGTTTACATTACGATTCTTTGTGTTATACAGTTGATGGGTTTCTTTTCTTTCTTA
ATTTCTTTAAAAAATAGAGATGGGGTCTTACTATATTACCCAGGCTGGTCTTGAAGTC
CTGGGCTCAAGTGATCTTCCTGTCTCAGCCTACCAAGTAGCTGAGACTATAGGTGCAA
AAAAGCCACTATACCTGGCTAGTTTACAGGTTTTAACAAATGCATTATGCCACGTATC
CATTATTACAGGATCACACAAGATATTTTCATTACCCTGAAAGCATCCCTGTGTTCCAC
CAATTCATCCTGCCTCCATGAGCCGCTGGCAACCACTGATCTCTATAGTTTTGCCTTTT
CTAAAATGTCATATAATTGGAATCATACAGTCTGTAGCATTTTCAGACTAGCTTTTAAA
ATTTGGCAATATGCATTTAAGGTTCCTCCTTAAATGTGAAGGGCATAGCCAATGTGGC
TTGATAGCTCATTTCTTTTTATTGGTGAATATTTCATTGTCTGGATGTTCCACAGTTTGT
TTATCCATTCACCTATTCAATTTGCTTTTTTTCTGTGTATCTATCACTAATTCAATACTG
G

Uppercase nucleotides in bold = the 5' and 3' *CLYBL* gene homology arms; uppercase bold underlined nucleotides = splice acceptor sequence; uppercase italic nucleotides = linker; lowercase bold underlined nucleotides = T2A sequence; uppercase bold italic nucleotides = puromycin resistance gene; lowercase bold italic nucleotides = bGH polyA sequence; lowercase bold nucleotides = insulator sequences; uppercase underlined nucleotides = hU6 promoter; lower case nucleotides = CRISPRa gRNA sequence (*NEUROG2*-targeting); underlined lower case nucleotides = tcRNA sequence.
AAVS1-dCas9-VPR (SEQ ID NO: 23):

TGCTTTCTCTGACCAGCATTCTCTCCCCTGGGCCTGTGCCGCTTTCTGTCTGTAGCTTG
TGGCCTGGGTCACCTCTACGGCTGGCCCAGATCCTTCCCTGCCGCCTCCTTCAGGTTC
CGTCTTCCTCCACTCCCTCTTCCCCTTGCTCTCTGCTGTGTTGCTGCCCAAGGATGCTC
TTTCCGGAGCACTTCCTTCTCGGCGCTGCACCACGTGATGTCCTCTGAGCGGATCCTC
CCCGTGTCTGGGTCCTCTCCGGGCATCTCTCCTCCCTCACCCAACCCCATGCCGTCTT
CACTCGCTGGGTTCCCTTTTCCTTCTCCTTCTGGGGCCTGTGCCATCTCTCGTTTCTTA
GGATGGCCTTCTCCGACGGATGTCTCCCTTGCGTCCCGCCTCCCCTTCTTGTAGGCCT

GCATCATCACCGTTTTTCTGGACAACCCCAAAGTACCCCGTCTCCCTGGCTTTAGCCA
CCTCTCCATCCTCTTGCTTTCTTTGCCTGGACACCCCGTTCTCCTGTGGATTCGGGTCA
CCTCTCACTCCTTTCATTTGGGCAGCTCCCCTACCCCCCTTACCTCTCTAGTCTGTGCT
AGCTCTTCCAGCCCCCTGTCATGGCATCTTCCAGGGGTCCGAGAGCTCAGCTAGTCTT
CTTCCTCCAACCCGGGCCCCTATGTCCACTTCAGGACAGCATGTTTGCTGCCTCCAGG
GATCCTGTGTCCCCGAGCTGGGACCACCTTATATTCCCAGGGCCGGTTAATGTGGCT
CTGGTTCTGGGTACTTTTATCTGTCCCCTCCACCCCACAGTGGGGCAAGCTTCTGACC
TCTTCTCTTCCTCCCACAGGGCCTCGAGAGATCTGGCAGCGGAgagggcagaggaagtcttct
aacatgcggtgacgtggaggagaatcccggccctAGGCTCGAGATGACCGAGTACAAGCCCACGG
TGCGCCTCGCCACCCGCGACGACGTCCCCAGGGCCGTACGCACCCTCGCCGCCGCG
TTCGCCGACTACCCCGCCACGCGCCACACCGTCGATCCGGACCGCCACATCGAGCG
GGTCACCGAGCTGCAAGAACTCTTCCTCACGCGCGTCGGGCTCGACATCGGCAAGGT
GTGGGTCGCGGACGACGGCGCCGCGGTGGCGGTCTGGACCACGCCGGAGAGCGTC
GAAGCGGGGGCGGTGTTCGCCGAGATCGGCCCGCGCATGGCCGAGTTGAGCGGTTC
CCGGCTGGCCGCGCAGCAACAGATGGAAGGCCTCCTGGCGCCGCACCGGCCCAAG
GAGCCCGCGTGGTTCCTGGCCACCGTCGGCGTCTCGCCCGACCACCAGGGCAAGGG
TCTGGGCAGCGCCGTCGTGCTCCCCGGAGTGGAGGCGGCCGAGCGCGCCGGGGTG
CCCGCCTTCCTGGAGACCTCCGCGCCCCGCAACCTCCCCTTCTACGAGCGGCTCGGC
TTCACCGTCACCGCCGACGTCGAGGTGCCCGAAGGACCGCGCACCTGGTGCATGAC
CCGCAAGCCCGGTGCCTGATCTAGAGGGCCCGTTTAAACCCGCTGATCAGCCTCGAct
gtgccttctagttgccagccatctgttgtttgcccctcccccgtgccttccttgaccctggaaggtgccactcccactgtc
ctttcctaataaaatgaggaaattgcatcgcattgtctgagtaggtgtcattctattctggggggtggggtggggcagga
cagcaagggggaggattgggaagacaatagcaggcatgctggggatgcggtgggctctatggTCACTCGAGT
TTACTCCCTATCAGTGATAGAGAACGTATGAAGAGTTTACTCCCTATCAGTGATAGAGAA
CGTATGCAGACTTTACTCCCTATCAGTGATAGAGAACGTATAAGGAGTTTACTCCCTATC
AGTGATAGAGAACGTATGACCAGTTTACTCCCTATCAGTGATAGAGAACGTATCTACAG
TTTACTCCCTATCAGTGATAGAGAACGTATATCCAGTTTACTCCCTATCAGTGATAGAGA
ACGTATGTCGAGGTAGGCGTGTACGGTGGGCGCCTATAAAAGCAGAGCTCGTTTAGTG
AACCGTCAGATCGCCTGGAGCAATTCCACAACACTTTTGTCTTATACTTAGTAGAAGGA
GAGGCCACCATGGCCccaaagaagaagcggaaggtcGGTATCCACGGAGTCCCAGCAGCCga
caagaagtacagcatcggcctggccatcggcaccaactctgtgggctgggccgtgatcaccgacgagtacaaggtgcccagc
aagaaattcaaggtgctgggcaacaccgaccggcacagcatcaagaagaacctgatcggcgccctgctgttcgacagcgga
gaaacagccgaggccacccggctgaagagaaccgccagaagaagatacaccagacggaagaaccggatctgctatctgc
aagagatcttcagcaacgagatggccaaggtggacgacagcttcttccacagactggaagagtccttcctggtggaagaggat
aagaagcacgagcggcaccccatcttcggcaacatcgtggacgaggtggcctaccacgagaagtaccccaccatctaccac
ctgagaaagaaactggtggacagcaccgacaaggccgacctgcggctgatctatctggccctggcccacatgatcaagttccg
gggccacttcctgatcgagggcgacctgaaccccgacaacagcgacgtggacaagctgttcatccagctggtgcagacctaca

accagctgttcgaggaaaaccccatcaacgccagcggcgtggacgccaaggccatcctgtctgccagactgagcaagagca
gacggctggaaaatctgatcgcccagctgcccggcgagaagaagaatggcctgttcggcaacctgattgccctgagcctgggc
ctgacccccaacttcaagagcaacttcgacctggccgaggatgccaaactgcagctgagcaaggacacctacgacgacgac
ctggacaacctgctggcccagatcggcgaccagtacgccgacctgtttctggccgccaagaacctgtccgacgccatcctgctg
agcgacatcctgagagtgaacaccgagatcaccaaggcccccctgagcgcctctatgatcaagagatacgacgagcaccac
caggacctgaccctgctgaaagctctcgtgcggcagcagctgcctgagaagtacaaagagattttcttcgaccagagcaagaa
cggctacgccggctacatcgatggcggagccagccaggaagagttctacaagttcatcaagcccatcctggaaaagatggac
ggcaccgaggaactgctcgtgaagctgaacagagaggacctgctgcggaagcagcggaccttcgacaacggcagcatccc
ccaccagatccacctgggagagctgcacgccattctgcggcggcaggaagatttttacccattcctgaaggacaaccgggaaa
agatcgagaagatcctgaccttccgcatcccctactacgtgggccctctggccaggggaaacagcagattcgcctggatgacca
gaaagagcgaggaaaccatcaccccctggaacttcgaggaagtggtggacaagggcgccagcgcccagagcttcatcgag
cggatgaccaacttcgataagaacctgcccaacgagaaggtgctgcccaagcacagcctgctgtacgagtacttcaccgtgta
caacgagctgaccaaagtgaaatacgtgaccgagggaatgagaaagcccgccttcctgagcggcgagcagaaaaaagcc
atcgtggacctgctgttcaagaccaaccggaaagtgaccgtgaagcagctgaaagaggactacttcaagaaaatcgagtgctt
cgactccgtggaaatctccggcgtggaagatcggttcaacgcctccctgggcacataccacgatctgctgaaaattatcaagga
caaggacttcctggacaatgaggaaaacgaggacattctggaagatatcgtgctgaccctgacactgtttgaggacagagaga
tgatcgaggaacggctgaaaacctatgcccacctgttcgacgacaaagtgatgaagcagctgaagcggcggagatacaccg
gctggggcaggctgagccggaagctgatcaacggcatccgggacaagcagtccggcaagacaatcctggatttcctgaagtc
cgacggcttcgccaacagaaacttcatgcagctgatccacgacgacagcctgacctttaaagaggacatccagaaagcccag
gtgtccggccagggcgatagcctgcacgagcacattgccaatctggccggcagccccgccattaagaagggcatcctgcaga
cagtgaaggtggtggacgagctcgtgaaagtgatgggccggcacaagcccgagaacatcgtgatcgaaatggccagagag
aaccagaccacccagaagggacagaagaacagccgcgagagaatgaagcggatcgaagagggcatcaaagagctggg
cagccagatcctgaaagaacaccccgtggaaaacacccagctgcagaacgagaagctgtacctgtactacctgcagaatgg
gcgggatatgtacgtggaccaggaactggacatcaaccggctgtccgactacgatgtggacgctatcgtgcctcagagctttctg
aaggacgactccatcgataacaaagtgctgactcggagcgacaagaaccggggcaagagcgacaacgtgccctccgaag
aggtcgtgaagaagatgaagaactactggcgccagctgctgaatgccaagctgattacccagaggaagttcgacaatctgacc
aaggccgagagaggcggcctgagcgaactggataaggccggcttcatcaagagacagctggtggaaacccggcagatcac
aaagcacgtggcacagatcctggactcccggatgaacactaagtacgacgagaacgacaaactgatccgggaagtgaaagt
gatcaccctgaagtccaagctggtgtccgatttccggaaggatttccagttttacaaagtgcgcgagatcaacaactaccaccac
gcccacgacgcctacctgaacgccgtcgtgggaaccgccctgatcaaaaagtaccctaagctggaaagcgagttcgtgtacg
gcgactacaaggtgtacgacgtgcggaagatgatcgccaagagcgagcaggaaatcggcaaggctaccgccaagtacttctt
ctacagcaacatcatgaactttttcaagaccgagattaccctggccaacggcgagatccggaagcggcctctgatcgagacaa
acggcgaaacaggcgagatcgtgtgggataagggccgggactttgccaccgtgcggaaagtgctgtctatgccccaagtgaat
atcgtgaaaaagaccgaggtgcagacaggcggcttcagcaaagagtctatcctgcccaagaggaacagcgacaagctgatc
gccagaaagaaggactgggaccctaagaagtacggcggcttcgacagccccaccgtggcctattctgtgctggtggtggccaa
agtggaaaagggcaagtccaagaaactgaagagtgtgaaagagctgctggggatcaccatcatggaaagaagcagcttcga
gaagaatcccatcgactttctggaagccaagggctacaaagaagtgaaaaaggacctgatcatcaagctgcctaagtactccc

tgttcgagctggaaaacggccggaagagaatgctggcctctgccggcgaactgcagaagggaaacgaactggccctgccctc
caaatatgtgaacttcctgtacctggccagccactatgagaagctgaagggctcccccgaggataatgagcagaaacagctgtt
tgtggaacagcacaaacactacctggacgagatcatcgagcagatcagcgagttctccaagagagtgatcctggccgacgct
aatctggacaaggtgctgagcgcctacaacaagcacagagacaagcctatcagagagcaggccgagaatatcatccacctg
tttaccctgaccaatctgggagcccctgccgccttcaagtactttgacaccaccatcgaccggaagaggtacaccagcaccaaa
gaggtgctggacgccaccctgatccaccagagcatcaccggcctgtacgagacacggatcgacctgtctcagctgggaggcg
acgcctatccctatgacgtgcccgattatgccagcctgGGCAGCGGCTCCcccaagaaaaaacgcaaggtggaaa
gcggctctgagactcccggcacatccgaaagcgcaacccctgagtctgaggccagcggttccggacgggctgacgcattgga
cgattttgatctggatatgctgggaagtgacgccctcgatgattttgaccttgacatgcttggttcggatgcccttgatgactttgacctc
gacatgctcggcagtgacgcccttgatgatttcgacctggacatgctgattaactctagaagttccggatctccgaaaaagaaac
gcaaagttggtagccagtacctgcccgacaccgacgaccggcaccggatcgaggaaaagcggaagcggacctacgagac
attcaagagcatcatgaagaagtcccccttcagcggccccaccgaccctagacctccacctagaagaatcgccgtgcccagca
gatccagcgccagcgtgccaaaacctgccccccagccttaccccttcaccagcagcctgagcaccatcaactacgacgagttc
cctaccatggtgttccccagcggccagatctctcaggcctctgctctggctccagcccctcctcaggtgctgcctcaggctcctgctc
ctgcaccagctccagccatggtgtctgcactggctcaggcaccagcacccgtgcctgtgctggctcctggacctccacaggctgt
ggctccaccagcccctaaacctacacaggccggcgagggcacactgtctgaagctctgctgcagctgcagttcgacgacgag
gatctgggagccctgctgggaaacagcaccgatcctgccgtgttcaccgacctggccagcgtggacaacagcgagttccagca
gctgctgaaccagggcatccctgtggcccctcacaccaccgagcccatgctgatggaataccccgaggccatcacccggctcg
tgacaggcgctcagaggcctcctgatccagctcctgcccctctgggagcaccaggcctgcctaatggactgctgtctggcgacg
aggacttcagctctatcgccgatatggatttctcagccttgctgggctctggcagcggcagccgggattccagggaagggatgtttt
tgccgaagcctgaggccggctccgctattagtgacgtgtttgagggccgcgaggtgtgccagccaaaacgaatccggccatttc
atcctccaggaagtccatgggccaaccgcccactccccgccagcctcgcaccaacaccaaccggtccagtacatgagccagt
cgggtcactgaccccggcaccagtccctcagccactggatccagcgcccgcagtgactcccgaggccagtcacctgttggagg
atcccgatgaagagacgagccaggctgtcaaagcccttcgggagatggccgatactgtgattccccagaaggaagaggctgc
aatctgtggccaaatggacctttcccatccgcccccaaggggccatctggatgagctgacaaccacacttgagtccatgaccga
ggatctgaacctggactcacccctgaccccggaattgaacgagattctggataccttcctgaacgacgagtgcctcttgcatgcca
tgcatatcagcacaggactgtccatcttcgacacatctctgttttaaCCGGGGATCCTCTAGTCAGCTGACGCG
TGCTAGCGCGGCCGCATCGATAAGCTTGTCGACGATATCTCTAGAGGATCATAATCAG
CCATACCACATTTGTAGAGGTTTTACTTGCTTTAAAAAACCTCCCACACCTCCCCCTGAA
CCTGAAACATAAAATGAATGCAATTGTTGTTGTTaacttgtttattgcagcttataatggttacaaataaagca
atagcatcacaaatttcacaaataaagcatttttttcactgcCTTGACAGTACTCTTAAGTCGACTTACTAGGG
ACAGGATTGGTGACAGAAAAGCCCCATCCTTAGGCCTCCTCCTTCCTAGTCTCCTGAT
ATTGGGTCTAACCCCCACCTCCTGTTAGGCAGATTCCTTATCTGGTGACACACCCCCA
TTTCCTGGAGCCATCTCTCTCCTTGCCAGAACCTCTAAGGTTTGCTTACGATGGAGCC
AGAGAGGATCCTGGGAGGGAGAGCTTGGCAGGGGGTGGGAGGGAAGGGGGGGATG
CGTGACCTGCCCGGTTCTCAGTGGCCACCCTGCGCTACCCTCTCCCAGAACCTGAGC
TGCTCTGACGCGGCTGTCTGGTGCGTTTCACTGATCCTGGTGCTGCAGCTTCCTTACA

CTTCCCAAGAGGAGAAGCAGTTTGGAAAAACAAAATCAGAATAAGTTGGTCCTGAGT
TCTAACTTTGGCTCTTCACCTTTCTAGTCCCCAATTTATATTGTTCCTCCGTGCGTCAGT
TTTACCTGTGAGATAAGGCCAGTAGCCAGCCCCGTCCTGGCAGGGCTGTGGTGAGGA
GGGGGGTGTCCGTGTGGAAAACTCCCTTTGTGAGAATGGTGCGTCCTAGGTGTTCAC
CAGGTCGTGGCCGCCTCTACTCCCTTTCTCTTTCTCCATCCTTCTTTCCTTAAAGAGTC
CCCAGTGCTATCTGGGACATATTCCTCCGCCCAGAGCAGGGTCCCGCTTCCCTAAGG
CCCTGCTCTGGGCTTCTGGGTTTGAGTCCTTGGCAAGCCCAGGAGAGGCGCTCAGGC
TTCCCTGTCCCCCTTCCTCGTCCACCATCTCATGCCCCTGGCTCTCCTGCCCCTTCCCT
ACAGGGGTTCCTGGCTCTGCTCT

Uppercase nucleotides in bold = the 5' and 3' *AAVS1* gene homology arms; uppercase bold underlined nucleotides = splice acceptor sequence; uppercase italic nucleotides = linker; lowercase bold underlined nucleotides = T2A sequence; uppercase bold italic nucleotides = puromycin resistance gene; lowercase bold italic nucleotides = bGH polyA sequence; lowercase bold nucleotides = nuclear localisation sequence; uppercase underlined nucleotides = TRE3G promoter; lower case nucleotides = dCas9 sequence; underlined lower case nucleotides = VPR sequence; lowercase italic nucleotides = SV40 polyA sequence.
AAVS1-Csy4-dCas9-VPR (SEQ ID NO: 24):

TGCTTTCTCTGACCAGCATTCTCTCCCCTGGGCCTGTGCCGCTTTCTGTCTGTAGCTTG
TGGCCTGGGTCACCTCTACGGCTGGCCCAGATCCTTCCCTGCCGCCTCCTTCAGGTTC
CGTCTTCCTCCACTCCCTCTTCCCCTTGCTCTCTGCTGTGTTGCTGCCCAAGGATGCTC
TTTCCGGAGCACTTCCTTCTCGGCGCTGCACCACGTGATGTCCTCTGAGCGGATCCTC
CCCGTGTCTGGGTCCTCTCCGGGCATCTCTCCTCCCTCACCCAACCCCATGCCGTCTT
CACTCGCTGGGTTCCCTTTTCCTTCTCCTTCTGGGGCCTGTGCCATCTCTCGTTTCTTA
GGATGGCCTTCTCCGACGGATGTCTCCCTTGCGTCCCGCCTCCCCTTCTTGTAGGCCT
GCATCATCACCGTTTTTCTGGACAACCCCAAAGTACCCCGTCTCCCTGGCTTTAGCCA
CCTCTCCATCCTCTTGCTTTCTTTGCCTGGACACCCCGTTCTCCTGTGGATTCGGGTCA
CCTCTCACTCCTTTCATTTGGGCAGCTCCCCTACCCCCCTTACCTCTCTAGTCTGTGCT
AGCTCTTCCAGCCCCCTGTCATGGCATCTTCCAGGGGTCCGAGAGCTCAGCTAGTCTT
CTTCCTCCAACCCGGGCCCCTATGTCCACTTCAGGACAGCATGTTTGCTGCCTCCAGG
GATCCTGTGTCCCCGAGCTGGGACCACCTTATATTCCCAGGGCCGGTTAATGTGGCT
CTGGTTCTGGGTACTTTTATCTGTCCCCTCCACCCCACAGTGGGGCAAGCTTCTGACC
TCTTCTCTTCCTCCCACAGGGCCTCGAGAGATCTGGCAGCGGAgagggcagaggaagtcttct
aacatgcggtgacgtggaggagaatcccggccctAGGCTCGAGATGACCGAGTACAAGCCCACGG
TGCGCCTCGCCACCCGCGACGACGTCCCCAGGGCCGTACGCACCCTCGCCGCCGCG
TTCGCCGACTACCCCGCCACGCGCCACACCGTCGATCCGGACCGCCACATCGAGCG

GGTCACCGAGCTGCAAGAACTCTTCCTCACGCGCGTCGGGCTCGACATCGGCAAGGT
GTGGGTCGCGGACGACGGCGCCGCGGTGGCGGTCTGGACCACGCCGGAGAGCGTC
GAAGCGGGGGCGGTGTTCGCCGAGATCGGCCCGCGCATGGCCGAGTTGAGCGGTTC
CCGGCTGGCCGCGCAGCAACAGATGGAAGGCCTCCTGGCGCCGCACCGGCCCAAG
GAGCCCGCGTGGTTCCTGGCCACCGTCGGCGTCTCGCCCGACCACCAGGGCAAGGG
TCTGGGCAGCGCCGTCGTGCTCCCCGGAGTGGAGGCGGCCGAGCGCGCCGGGGTG
CCCGCCTTCCTGGAGACCTCCGCGCCCCGCAACCTCCCCTTCTACGAGCGGCTCGGC
TTCACCGTCACCGCCGACGTCGAGGTGCCCGAAGGACCGCGCACCTGGTGCATGAC
CCGCAAGCCCGGTGCCTGATCTAGAGGGCCCGTTTAAACCCGCTGATCAGCCTCGAct
gtgccttctagttgccagccatctgttgtttgcccctcccccgtgccttccttgaccctggaaggtgccactcccactgtc
ctttcctaataaaatgaggaaattgcatcgcattgtctgagtaggtgtcattctattctggggggtggggtggggcagga
cagcaagggggaggattgggaagacaatagcaggcatgctggggatgcggtgggctctatggTCACTCGAGT
TTACTCCCTATCAGTGATAGAGAACGTATGAAGAGTTTACTCCCTATCAGTGATAGAGAA
CGTATGCAGACTTTACTCCCTATCAGTGATAGAGAACGTATAAGGAGTTTACTCCCTATC
AGTGATAGAGAACGTATGACCAGTTTACTCCCTATCAGTGATAGAGAACGTATCTACAG
TTTACTCCCTATCAGTGATAGAGAACGTATATCCAGTTTACTCCCTATCAGTGATAGAGA
ACGTATGTCGAGGTAGGCGTGTACGGTGGGCGCCTATAAAAGCAGAGCTCGTTTAGTG
AACCGTCAGATCGCCTGGAGCAATTCCACAACACTTTTGTCTTATACTTAGTAGAAGGA
GAGGCCACCAATGGGTGACCACTATCTCGACATCAGATTGCGACCGGATCCCGAGTTC
CCTCCTGCGCAACTGATGTCCGTACTGTTTGGGAAATTGCACCAAGCCCTCGTCGCGC
AAGGCGGCGATAGGATAGGCGTTAGTTTTCCTGATCTCGATGAGTCAAGATCCCGACT
CGGTGAGCGCCTGCGAATACATGCTTCAGCAGATGACCTCAGGGCACTTCTGGCTCGG
CCGTGGTTGGAGGGTCTTAGGGACCATTTGCAATTTGGTGAACCCGCCGTCGTCCCGC
ATCCAACCCCATATCGGCAAGTAAGCAGAGTCCAGGCTAAATCAAACCCCGAGAGATT
GAGGCGGCGGCTTATGCGACGCCACGATCTCTCCGAGGAAGAGGCTCGAAAACGGAT
ACCGGACACGGTGGCCAGGGCACTCGACCTGCCATTTGTAACCCTTCGCTCCCAGTCC
ACTGGTCAACATTTCCGATTGTTTATTAGACACGGTCCTTTGCAGGTGACTGCTGAGGA
GGGGGGATTTACATGCTACGGACTCAGCAAAGGGGGTTTTGTCCCGTGGTTTTgagggc
agaggaagtctgttaacatgcggtgacgtcgaggagaatcctggcccaATGGGCCccaaagaagaagcggaag
gtcGGTATCCACGGAGTCCCAGCAGCCgacaagaagtacagcatcggcctggccatcggcaccaactctgtg
ggctgggccgtgatcaccgacgagtacaaggtgcccagcaagaaattcaaggtgctgggcaacaccgaccggcacagcatc
aagaagaacctgatcggcgccctgctgttcgacagcggagaaacagccgaggccacccggctgaagagaaccgccagaa
gaagatacaccagacggaagaaccggatctgctatctgcaagagatcttcagcaacgagatggccaaggtggacgacagctt
cttccacagactggaagagtccttcctggtggaagaggataagaagcacgagcggcaccccatcttcggcaacatcgtggacg
aggtggcctaccacgagaagtaccccaccatctaccacctgagaaagaaactggtggacagcaccgacaaggccgacctg
cggctgatctatctggccctggcccacatgatcaagttccggggccacttcctgatcgagggcgacctgaaccccgacaacagc
gacgtggacaagctgttcatccagctggtgcagacctacaaccagctgttcgaggaaaaccccatcaacgccagcggcgtgg

acgccaaggccatcctgtctgccagactgagcaagagcagacggctggaaaatctgatcgcccagctgcccggcgagaaga
agaatggcctgttcggcaacctgattgccctgagcctgggcctgacccccaacttcaagagcaacttcgacctggccgaggatg
ccaaactgcagctgagcaaggacacctacgacgacgacctggacaacctgctggcccagatcggcgaccagtacgccgac
ctgtttctggccgccaagaacctgtccgacgccatcctgctgagcgacatcctgagagtgaacaccgagatcaccaaggcccc
cctgagcgcctctatgatcaagagatacgacgagcaccaccaggacctgaccctgctgaaagctctcgtgcggcagcagctgc
ctgagaagtacaaagagattttcttcgaccagagcaagaacggctacgccggctacatcgatggcggagccagccaggaag
agttctacaagttcatcaagcccatcctggaaaagatggacggcaccgaggaactgctcgtgaagctgaacagagaggacct
gctgcggaagcagcggaccttcgacaacggcagcatcccccaccagatccacctgggagagctgcacgccattctgcggcg
gcaggaagatttttacccattcctgaaggacaaccgggaaaagatcgagaagatcctgaccttccgcatcccctactacgtggg
ccctctggccaggggaaacagcagattcgcctggatgaccagaaagagcgaggaaaccatcaccccctggaacttcgagga
agtggtggacaagggcgccagcgcccagagcttcatcgagcggatgaccaacttcgataagaacctgcccaacgagaaggt
gctgcccaagcacagcctgctgtacgagtacttcaccgtgtacaacgagctgaccaaagtgaaatacgtgaccgagggaatg
agaaagcccgccttcctgagcggcgagcagaaaaaagccatcgtggacctgctgttcaagaccaaccggaaagtgaccgtg
aagcagctgaaagaggactacttcaagaaaatcgagtgcttcgactccgtggaaatctccggcgtggaagatcggttcaacgc
ctccctgggcacataccacgatctgctgaaaattatcaaggacaaggacttcctggacaatgaggaaaacgaggacattctgg
aagatatcgtgctgaccctgacactgtttgaggacagagagatgatcgaggaacggctgaaaacctatgcccacctgttcgacg
acaaagtgatgaagcagctgaagcggcggagatacaccggctggggcaggctgagccggaagctgatcaacggcatccgg
gacaagcagtccggcaagacaatcctggatttcctgaagtccgacggcttcgccaacagaaacttcatgcagctgatccacga
cgacagcctgacctttaaagaggacatccagaaagcccaggtgtccggccagggcgatagcctgcacgagcacattgccaat
ctggccggcagccccgccattaagaagggcatcctgcagacagtgaaggtggtggacgagctcgtgaaagtgatgggccgg
cacaagcccgagaacatcgtgatcgaaatggccagagagaaccagaccacccagaagggacagaagaacagccgcga
gagaatgaagcggatcgaagagggcatcaaagagctgggcagccagatcctgaaagaacaccccgtggaaaacacccag
ctgcagaacgagaagctgtacctgtactacctgcagaatgggcgggatatgtacgtggaccaggaactggacatcaaccggct
gtccgactacgatgtggacgctatcgtgcctcagagctttctgaaggacgactccatcgataacaaagtgctgactcggagcgac
aagaaccggggcaagagcgacaacgtgccctccgaagaggtcgtgaagaagatgaagaactactggcgccagctgctgaa
tgccaagctgattacccagaggaagttcgacaatctgaccaaggccgagagaggcggcctgagcgaactggataaggccgg
cttcatcaagagacagctggtggaaacccggcagatcacaaagcacgtggcacagatcctggactcccggatgaacactaag
tacgacgagaacgacaaactgatccgggaagtgaaagtgatcaccctgaagtccaagctggtgtccgatttccggaaggatttc
cagttttacaaagtgcgcgagatcaacaactaccaccacgcccacgacgcctacctgaacgccgtcgtgggaaccgccctgat
caaaaagtaccctaagctggaaagcgagttcgtgtacggcgactacaaggtgtacgacgtgcggaagatgatcgccaagagc
gagcaggaaatcggcaaggctaccgccaagtacttcttctacagcaacatcatgaactttttcaagaccgagattaccctggcca
acggcgagatccggaagcggcctctgatcgagacaaacggcgaaacaggcgagatcgtgtgggataagggccgggactttg
ccaccgtgcggaaagtgctgtctatgccccaagtgaatatcgtgaaaaagaccgaggtgcagacaggcggcttcagcaaaga
gtctatcctgcccaagaggaacagcgacaagctgatcgccagaaagaaggactgggaccctaagaagtacggcggcttcga
cagccccaccgtggcctattctgtgctggtggtggccaaagtggaaaagggcaagtccaagaaactgaagagtgtgaaagag
ctgctggggatcaccatcatggaaagaagcagcttcgagaagaatcccatcgactttctggaagccaagggctacaaagaagt
gaaaaaggacctgatcatcaagctgcctaagtactccctgttcgagctggaaaacggccggaagagaatgctggcctctgccg

gcgaactgcagaagggaaacgaactggccctgccctccaaatatgtgaacttcctgtacctggccagccactatgagaagctg
aagggctcccccgaggataatgagcagaaacagctgtttgtggaacagcacaaacactacctggacgagatcatcgagcag
atcagcgagttctccaagagagtgatcctggccgacgctaatctggacaaggtgctgagcgcctacaacaagcacagagaca
agcctatcagagagcaggccgagaatatcatccacctgtttaccctgaccaatctgggagcccctgccgccttcaagtactttgac
accaccatcgaccggaagaggtacaccagcaccaaagaggtgctggacgccaccctgatccaccagagcatcaccggcct
gtacgagacacggatcgacctgtctcagctgggaggcgacgcctatccctatgacgtgcccgattatgccagcctgGGCAG
CGGCTCCCCCAAGAAAAAACGCAAGGTGGAAagcggctctgagactcccggcacatccgaaagcgcaa
cccctgagtctgaggccagcggttccggacgggctgacgcattggacgattttgatctggatatgctgggaagtgacgccctcga
tgattttgaccttgacatgcttggttcggatgcccttgatgactttgacctcgacatgctcggcagtgacgcccttgatgatttcgacctg
gacatgctgattaactctagaagttccggatctccgaaaaagaaacgcaaagttggtagccagtacctgcccgacaccgacga
ccggcaccggatcgaggaaaagcggaagcggacctacgagacattcaagagcatcatgaagaagtcccccttcagcggcc
ccaccgaccctagacctccacctagaagaatcgccgtgcccagcagatccagcgccagcgtgccaaaacctgccccccagc
cttaccccttcaccagcagcctgagcaccatcaactacgacgagttccctaccatggtgttccccagcggccagatctctcaggc
ctctgctctggctccagcccctcctcaggtgctgcctcaggctcctgctcctgcaccagctccagccatggtgtctgcactggctcag
gcaccagcacccgtgcctgtgctggctcctggacctccacaggctgtggctccaccagcccctaaacctacacaggccggcga
gggcacactgtctgaagctctgctgcagctgcagttcgacgacgaggatctgggagccctgctgggaaacagcaccgatcctg
ccgtgttcaccgacctggccagcgtggacaacagcgagttccagcagctgctgaaccagggcatccctgtggcccctcacacc
accgagcccatgctgatggaataccccgaggccatcacccggctcgtgacaggcgctcagaggcctcctgatccagctcctgc
ccctctgggagcaccaggcctgcctaatggactgctgtctggcgacgaggacttcagctctatcgccgatatggatttctcagcctt
gctgggctctggcagcggcagccgggattccagggaagggatgtttttgccgaagcctgaggccggctccgctattagtgacgt
gtttgagggccgcgaggtgtgccagccaaaacgaatccggccatttcatcctccaggaagtccatgggccaaccgcccactcc
ccgccagcctcgcaccaacaccaaccggtccagtacatgagccagtcgggtcactgaccccggcaccagtccctcagccact
ggatccagcgcccgcagtgactcccgaggccagtcacctgttggaggatcccgatgaagagacgagccaggctgtcaaagc
ccttcgggagatggccgatactgtgattccccagaaggaagaggctgcaatctgtggccaaatggacctttcccatccgccccca
aggggccatctggatgagctgacaaccacacttgagtccatgaccgaggatctgaacctggactcacccctgaccccggaatt
gaacgagattctggataccttcctgaacgacgagtgcctcttgcatgccatgcatatcagcacaggactgtccatcttcgacacat
ctctgttttaaCCGGGGATCCTCTAGTCAGCTGACGCGTGCTAGCGCGGCCGCATCGATAAG
CTTGTCGACGATATCTCTAGAGGATCATAATCAGCCATACCACATTTGTAGAGGTTTTAC
TTGCTTTAAAAAACCTCCCACACCTCCCCCTGAACCTGAAACATAAAATGAATGCAATTG
TTGTTGTTaacttgtttattgcagcttataatggttacaaataaagcaatagcatcacaaatttcacaaataaagcatttttttcac
tgcCTTGACAGTACTCTTAAGTCGACTTACTAGGGACAGGATTGGTGACAGAAAAGCCC
CATCCTTAGGCCTCCTCCTTCCTAGTCTCCTGATATTGGGTCTAACCCCCACCTCCTGT
TAGGCAGATTCCTTATCTGGTGACACACCCCCATTTCCTGGAGCCATCTCTCTCCTTG
CCAGAACCTCTAAGGTTTGCTTACGATGGAGCCAGAGAGGATCCTGGGAGGGAGAG
CTTGGCAGGGGGTGGGAGGGAAGGGGGGGATGCGTGACCTGCCCGGTTCTCAGTGG
CCACCCTGCGCTACCCTCTCCCAGAACCTGAGCTGCTCTGACGCGGCTGTCTGGTGC
GTTTCACTGATCCTGGTGCTGCAGCTTCCTTACACTTCCCAAGAGGAGAAGCAGTTTG

GAAAAACAAAATCAGAATAAGTTGGTCCTGAGTTCTAACTTTGGCTCTTCACCTTTCTA
GTCCCCAATTTATATTGTTCCTCCGTGCGTCAGTTTTACCTGTGAGATAAGGCCAGTA
GCCAGCCCCGTCCTGGCAGGGCTGTGGTGAGGAGGGGGGTGTCCGTGTGGAAAACT
CCCTTTGTGAGAATGGTGCGTCCTAGGTGTTCACCAGGTCGTGGCCGCCTCTACTCCC
TTTCTCTTTCTCCATCCTTCTTTCCTTAAAGAGTCCCCAGTGCTATCTGGGACATATTC
CTCCGCCCAGAGCAGGGTCCCGCTTCCCTAAGGCCCTGCTCTGGGCTTCTGGGTTTG
AGTCCTTGGCAAGCCCAGGAGAGGCGCTCAGGCTTCCCTGTCCCCCTTCCTCGTCCA
CCATCTCATGCCCCTGGCTCTCCTGCCCCTTCCCTACAGGGGTTCCTGGCTCTGCTCT

Uppercase nucleotides in bold = the 5' and 3' *AAVS1* gene homology arms; uppercase bold underlined nucleotides = splice acceptor sequence; uppercase italic nucleotides = linker; lowercase bold underlined nucleotides = T2A sequence; uppercase bold italic nucleotides = puromycin resistance gene; lowercase bold italic nucleotides = bGH polyA sequence; lowercase bold nucleotides = nuclear localisation sequence; uppercase underlined nucleotides = TRE3G promoter; uppercase grey highlighted nucleotides = Csy4 sequence; lower case nucleotides = dCas9 sequence; underlined lower case nucleotides = VPR sequence; lowercase italic nucleotides = SV40 polyA sequence.

## Claims

1. A method of forward programming an induced pluripotent stem cell (iPSC) into a somatic cell, said method comprising:
(i) targeted insertion of a gene encoding a transcriptional regulator protein into a first genomic safe harbour (GSH) site;
(ii) targeted insertion of an inducible cassette into a second GSH site, wherein said inducible cassette comprises a genetic sequence encoding a catalytically inactive programable nuclease protein and one or more transcription activator proteins operably linked to an inducible promoter, and wherein said inducible promoter is regulated by the transcriptional regulator protein;
(iii) targeted insertion of one or more guide RNA (gRNA) sequences into a third GSH site, wherein said one or more gRNA sequences are complementary to one or more transcription start sites (TSSs) of an endogenous lineage-specific factor gene and is operably linked to a constitutive promoter,
wherein the first, second and third GSH sites are different.

2. The method of claim 1, wherein the one or more gRNA sequences targets the catalytically inactive programmable nuclease protein and one or more transcription activator proteins to the TSS of the endogenous lineage-specific factor gene, and/or
wherein the one or more transcription activator proteins activates or increases transcription at the TSS of the endogenous lineage-specific factor gene.

3. The method of claim 1 or claim 2, wherein the one or more transcription activator proteins is fused to the catalytically inactive programable nuclease protein.

4. The method of any one of claims 1 to 3, wherein the one or more transcription activator proteins is selected from one or more of: VP16, VP64, p65, Rta, MS2 and HSF1, such as VP64, and/or
wherein the one or more transcription activator proteins is a combination of VP64, p65 and Rta or a combination of MS2, p65 and HSF1, such as wherein the transcription activator protein is a VP64-p65-Rta fusion protein or a MS2-p65-HSF1 fusion protein.

5. The method of any one of claims 1 to 4, wherein two or more gRNA sequences are inserted into the third GSH site, and wherein each of the gRNA sequences are complementary to alternative or different TSSs of the endogenous lineage-specific factor gene, or are complementary to more than one alternative sequences of the TSS of the endogenous lineage-specific factor gene.

6. The method of any one of claims 1 to 5, wherein the one or more gRNA sequences comprise an MS2 aptamer sequence, optionally wherein the MS2 aptamer sequence recruits an MS2-containing fusion protein to the TSS of the endogenous lineage-specific factor gene, such as an MS2-p65-HSF1 fusion protein.

7. The method of any one of claims 1 to 6, wherein the catalytically inactive programable nuclease protein is catalytically inactive Cas9, optionally comprising point mutations in the RuvCI and HNH nuclease domains, such as D10A and H840A compared to the wild-type sequence of Cas9.

8. The method of any one of claims 1 to 7, wherein the activity of the transcriptional regulator protein is controlled by an exogenous substance.

9. The method of any one of claims 1 to 8, wherein the transcriptional regulator protein is constitutively expressed.

10. The method of any one of claims 1 to 9, wherein the transcriptional regulator protein is selected from any one of: a tetracycline-responsive transcriptional activator protein (rtTa), a tetracycline repressor (TetR), VgEcR synthetic receptor, a cumate repressor (CymR) or a hybrid transcriptional regulator protein comprising a DNA binding domain from the yeast GAL4 protein, a truncated ligand binding domain from the human progesterone receptor and an activation domain from the human NF-κB,
optionally wherein the transcriptional regulator protein is rtTA and its activity is controlled by tetracycline of a derivative thereof, such as doxycycline, and optionally wherein the inducible promoter includes a tetracycline-response element (TRE).

11. The method of any one of claims 1 to 10, wherein the one or more gRNA sequences are complementary to the TSS of the endogenous *NEUROG2* gene and/or the endogenous *NEUROD1* gene, and wherein the iPSC is forward programmed into a neuron.

12. A method of forward programming an iPSC into a neuron, said method comprising:
(i) targeted insertion of a gene encoding a transcriptional regulator protein into a first genomic safe harbour (GSH) site, wherein the first GSH site is the ROSA26 locus;
(ii) targeted insertion of an inducible cassette into a second GSH site, wherein said inducible cassette comprises a genetic sequence encoding a catalytically inactive programmable nuclease protein and one or more transcription activator proteins operably linked to an inducible promoter, wherein said inducible promoter is regulated by the transcriptional regulator protein, and wherein the second GSH site is the AAVS1 locus; and
(iii) targeted insertion of one or more guide RNA (gRNA) sequences into a third GSH site, wherein said one or more gRNA sequences are complementary to one or more transcription start sites (TSSs) of the endogenous *NEUROG2* gene and/or the TSS of the endogenous *NEUROD1* gene and is operably linked to a constitutive promoter, and wherein the third GSH site is the CLYBL gene.

13. A cell obtained by the method of any one of claims 1 to 12.

14. A cell with a modified genome that comprises:
(i) an inserted genetic sequence encoding a transcriptional regulator protein at a first genomic safe harbour (GSH) site;
(ii) an inserted inducible cassette comprising a genetic sequence encoding a catalytically inactive programmable nuclease protein and one or more transcription activator proteins operably linked to an inducible promoter at a second GSH site, wherein said inducible promoter is regulated by the transcriptional regulator protein; and
(iii) an inserted genetic sequence encoding one or more gRNA sequences operably linked to a constitutive promoter at a third GSH site, wherein said one or more gRNA sequences are complementary to one or more TSS of an endogenous lineage-specific factor gene,
wherein the first, second and third GSH sites are different.

15. The cell of claim 13 or claim 14 for use in therapy, such as for the treatment of cancer, a neurological disorder, an inflammatory disease, an autoimmune disease and/or a chronic infectious disease.
